# EUROPEAN PATENT SPECIFICATION

(11) **EP 2 411 541 B1**
(45) Date of publication and mention of the grant of the patent: **11.02.2015**
(21) Application number: 10756913.9
(22) Date of filing: 26.03.2010
(51) Int. Cl.: C12Q 1/68, C12N 15/12, G01N 33/53

(54) **MARKERS RELATED TO AGE-RELATED MACULAR DEGENERATION AND USES THEREFOR**
MARKER IM ZUSAMMENHANG MIT ALTERBEDINGTER MAKULADEGENERATION UND IHRE VERWENDUNG
MARQUEURS ASSOCIÉS À LA DÉGÉNÉRESCENCE MACULAIRE LIÉE À L'ÂGE, ET UTILISATIONS AFFÉRENTES

(30) Priority: 27.03.2009 US 164245 P; 25.03.2010 US 317498 P
(43) Date of publication of application: 01.02.2012
(60) Divisional application: 14194664.0
(73) Proprietor: Tufts Medical Center, Inc., Boston, MA 02111 (US); The General Hospital Corporation, Boston, MA 02114 (US)
(72) Inventor: SEDDON, Johanna M., Boston Massachusetts 02108 (US); DALY, Mark, Arlington, Massachusetts 02474 (US)
(74) Representative: Fleuchaus, Andrea
(86) International application number: PCT/US2010/028834
(87) International publication number: WO 2010/111595

(56) References cited:
- WO-A2-2008/140793
- KLAVER C C W ET AL: "GENETIC ASSOCIATION OF APOLIPOPROTEIN E WITH AGE-RELATED MACULAR DEGENERATION", AMERICAN JOURNAL OF HUMAN GENETICS, AMERICAN SOCIETY OF HUMAN GENETICS, CHICAGO, IL, US, vol. 63, no. 1, 1 July 1998 (1998-07-01), pages 200-206, XP000911959, ISSN: 0002-9297, DOI: 10.1086/301901
- AFFYMETRIX: "Data Sheet Affymetrix(R) Genome-Wide Human SNP Array 6.0", INTERNET CITATION, 2007, pages 1-4, XP002525407, Retrieved from the Internet: URL:http://www.affymetrix.com/support/tech nical/datasheets/genomewide_snp6_datasheet .pdf [retrieved on 2009-04-10]
- R. V. SHOHET ET AL: "Hepatic Lipase (LIPC) Promoter Polymorphism in Men With Coronary Artery Disease : Allele Frequency and Effects on Hepatic Lipase Activity and Plasma HDL-C Concentrations", ARTERIOSCLEROSIS, THROMBOSIS, AND VASCULAR BIOLOGY, vol. 19, no. 8, 1 August 1999 (1999-08-01) , pages 1975-1978, XP55032878, ISSN: 1079-5642, DOI: 10.1161/01.ATV.19.8.1975
- KLEIN ROBERT J ET AL: "Complement factor H polymorphism in age-related macular degeneration", SCIENCE, AMERICAN ASSOCIATION FOR THE ADVANCEMENT OF SCIENCE, WASHINGTON, DC; US, vol. 308, no. 5720, 10 March 2005 (2005-03-10), pages 385-389, XP002391906, ISSN: 0036-8075, DOI: 10.1126/SCIENCE.1109557
- SPENCER ET AL.: 'Deletion of CFHR3 and CFHR1 genes in age-related macular degeneration' HUM MOL GENET vol. 17, no. 7, 15 December 2007, pages 971 - 977, XP008151867
- NG ET AL.: 'Multiple gene polymorphisms in the complement factor h gene are associated with exudative age-related macular degeneration in chinese' INVEST OPHTHALMOL VIS SCI vol. 49, no. 8, 17 April 2008, pages 3312 - 3317, XP002580199
- TSAI ET AL.: 'Interleukin gene polymorphisms in age-related macular degeneration' INVEST OPHTHALMOL VIS SCI. vol. 49, no. 2, February 2008, pages 693 - 698, XP008160279
- CHEN ET AL.: 'Association of HTRA1 polymorphism and bilaterality in advanced age-related macular degeneration' VISION RES vol. 48, no. 5, February 2008, pages 690 - 694, XP022475586
- FRANCIS ET AL.: 'Haplotypes in the complement factor H (CFH) gene: associations with drusen and advanced age-related macular degeneration' PLOS ONE vol. 2, no. 11, 28 November 2007, page E1197, XP008102569
- MORI ET AL.: 'Coding and noncoding variants in the CFH gene and cigarette smoking influence the risk of age-related macular degeneration in a Japanese population' INVEST OPHTHALMOL VIS SCI vol. 48, no. 11, November 2007, pages 5315 - 5319, XP008162666
- HUGHES GUY A ET AL: "LIPC Is A Possible Heritability Factor For Advanced Age-related Macular Degeneration", INVESTIGATIVE OPHTHALMOLOGY & VISUAL SCIENCE; ANNUAL MEETING OF THE ASSOCIATION-FOR-RESEARCH-IN-VISION-AND-OPH THALMOLOGY (ARVO), ASSOCIATION FOR RESEARCH IN VISION AND OPHTHALMOLOGY, US; FT LAUDERDALE, FL, USA, vol. 52, 1 January 2011 (2011-01-01), page 5263, XP008161819, ISSN: 0146-0404

## Description

### Background of the Invention

Age-related macular degeneration (AMD) is the most common geriatric eye disorder leading to blindness. Macular degeneration is responsible for visual handicap in what is estimated conservatively to be approximately 16 million individuals worldwide. Among the elderly, the overall prevalence is estimated between 5.7% and 30% depending on the definition of early AMD, and its differentiation from features of normal aging, a distinction that remains poorly understood.

Histopathologically, the hallmark of early neovascular AMD is accumulation of extracellular drusen and basal laminar deposit (abnormal material located between the plasma membrane and basal lamina of the retinal pigment epithelium) and basal linear deposit (material located between the basal lamina of the retinal pigment epithelium and the inner collageneous zone of Bruch's membrane). The end stage of AMD is characterized by a complete degeneration of the neurosensory retina and of the underlying retinal pigment epithelium in the macular area. Advanced stages of AMD can be subdivided into geographic atrophy and exudative AMD. Geographic atrophy is characterized by progressive atrophy of the retinal pigment epithelium. In exudative AMD the key phenomenon is the occurrence of choroidal neovascularisation (CNV). Eyes with CNV have varying degrees of reduced visual acuity, depending on location, size, type and age of the neovascular lesion. The development of choroidal neovascular membranes can be considered a late complication in the natural course of the disease possibly due to tissue disruption (Bruch's membrane) and decompensation of the underlying longstanding processes of AMD.

Many pathophysiological aspects as well as vascular and environmental risk factors are associated with a progression of the disease, but little is known about the etiology of AMD itself as well as about the underlying processes of complications like the occurrence of CNV. Family, twin, segregation, and case-control studies suggest an involvement of genetic factors in the etiology of AMD. The extent of heritability, number of genes involved, and mechanisms underlying phenotypic heterogeneity, however, are unknown. The search for genes and markers related to AMD faces challenges- onset is late in life, and there is usually only one generation available for studies. The parents of patients are often deceased, and the children are too young to manifest the disease. Generally, the heredity of late-onset diseases has been difficult to estimate because of the uncertainties of the diagnosis in previous generations and the inability to diagnose AMD among the children of an affected individual. Even in the absence of the ambiguities in the diagnosis of AMD in previous generations, the late onset of the condition itself, natural death rates, and small family sizes result in underestimation of genetic forms of AMD, and in overestimation of rates of sporadic disease. Moreover, the phenotypic variability is considerable, and it is conceivable that the currently used diagnostic entity of AMD in fact represents a spectrum of underlying conditions with various genetic and environmental factors involved.
Although several assessments of genetic association of particular polymorphisms and the risk to develop AMD have been published, such as in WO 2008 140793 disclosed a risk assessment for AMD due to the association of polymorphisms on some complement factors and in Klaver et al. 1998, (Am. J. Human Genetics Vol. 63, p. 200), who found a genetic correlation between apoE and AMD, there remains still a strong need for improved methods of diagnosing or prognosticating AMD or a susceptibility to AMD in subjects, as well as for evaluating and developing new methods of treatment. It is an object of the invention to identify inherited risk factors that suggest an increased risk in developing AMD or predicting the onset of more aggressive forms of the disease.

### Summary

The present invention is directed to methods that allow for improved diagnosis of AMD and susceptibility to AMD. The methods of the invention are directed to the discovery of genetic markers associated with lipid metabolism and metalloproteinase genes. These markers and polymorphisms are useful for diagnosing AMD or a susceptibility to AMD, for use as drug targets, for identifying therapeutic agents, and for determining the efficacy of and a subject's responsiveness to a therapeutic treatment.

In one aspect, the present invention is directed toward a method for diagnosing AMD or a susceptibility to AMD, a protective phenotype for AMD, or a neutral genotype for AMD, comprising detecting the presence or absence of a particular allele at a polymorphic site associated with lipid metabolism genes, where the allele is indicative of AMD or a susceptibility to AMD.

In one embodiment, the polymorphic site is a single nucleotide polymorphism associated with high density lipoprotein cholesterol (HDL-c) pathway genes, namely the polymorphic site is rs493258 (SEQ ID NO:1), where the cytidine polymorphism is indicative of AMD or susceptibility to AMD. In other embodiments, the polymorphic site is rs10468017 (SEQ ID NO:38), wherein the thymine polymorphism is indicative of AMD or susceptibility to AMD.

In another aspect, the present invention is directed toward a method for determining AMD risk in a patient, including: obtaining a patient sample, detecting an AMD marker in the patient sample further comprising determining the presence or absence of a particular allele at a polymorphic site associated with one or more metalloproteinase genes, wherein the allele indicates: a susceptibility for AMD, a protective phenotype for AMD or a neutral genotype for AMD, thereby indicating AMD risk in the patient. In one embodiment, the presence or absence of a particular allele is detected by a hybridization assay. In another embodiment, the presence or absence of a particular allele is determined using a microarray. In yet another embodiment, the presence or absence of a particular allele is determined using an antibody.

In one aspect, the present invention is directed toward a method for identifying a subject who is at risk or protected from developing AMD, including detecting the presence or absence of at least one at risk allele at rs493258 and one at risk allele at rs10468017; and detecting the presence or absence of at least one at risk allele or protective allele associated with LIPC gene; where a subject is not at risk if the subject is one of about 20% of the population with a less than about 1% risk of developing AMD, and the subject is at risk if the subject is one of about 1% of the population with a greater than about 50% risk of developing AMD.

In one aspect, the present application is directed toward a diagnostic system including: a diagnostic array, an array reader, an image processor, a database having data records and information records, a processor, and an information output; wherein the system compiles and processes patient data and outputs information relating to the statistical probability of the patient developing AMD.

In another aspect, the present application is directed toward a method of using the diagnostic system, including contacting a subject sample to the diagnostic array under high stringency hybridization conditions; inputting patient information into the system; and obtaining from the system information relating to the statistical probability of the patient developing AMD.

In yet another aspect, the present invention is directed toward a method for diagnosing AMD or a susceptibility to AMD in a subject comprising combining genetic risk with behavioral risk, wherein the genetic risk is determined by detecting the presence or absence of a particular allele at a polymorphic site associated with a lipid metabolism or metalloproteinase genes, wherein the allele is indicative of AMD or a susceptibility to AMD.

In one embodiment, the polymorphic site is rs493258 (SEQ ID NO: 1), where the cytidine polymorphism is indicative of AMD or susceptibility to AMD.

In one embodiment, the presence or absence of a particular allele is detected by a hybridization assay. In another embodiment, the presence or absence of a particular allele is determined using a microarray. In still another embodiment, the presence or absence of a particular allele is determined using an antibody.

In one embodiment, the behavioral risk is assessed by determining if the subject exhibits a behavior or trait selected from the group consisting of: obesity, smoking, vitamin and dietary supplement intake, use of alcohol or drugs, poor diet and a sedentary lifestyle. In another embodiment, the elevated BMI is used to determine obesity.

### Brief Description of the Drawings

FIG. 1A is a graph showing the distribution of effects from the discovery phase on analysis of 726,970 SNPs. The points coded in blue are loci from the previous AMD associated regions including CFH, ARMS2, CFI, C2, and C3.
FIG. 1B is a graph showing the distribution of P-values with the previously reported AMD associations removed, resulting in exclusion of 159 SNPs from this figure. The lambda of this distribution is approximately 1.04.
FIG. 2A is a graph showing the first two principle components for GWAS of AMD and MIGEN. The AMD samples are in red and the controls are in blue.
FIG. 2B is a graph showing the first two axes of variation for requiring 99% call rate for all SNPs. The AMD samples are in red and the MIGEN controls are in blue. The samples with a value greater than 0.02 on the second component have been excluded.
FIG. 3 is a graph showing 80% power to detect a biallelic CNV (copy number variation). Based on the sample size used in the CNV analysis of 459 controls and 838 cases the power was plotted to detect a bialleleic CNV (for example a simple deletion) at the p<0.001 level.
FIG 4. LIPC sequence analysis and retinal expression.
   (a) Phylogenetic tree of LIPC. TreeFam (Tree families database) analysis of the LIPC precursor revealed a large family of orthologs in diverse species, with a focus on vertebrates.
   (b) Protein analysis of adult bovine retinal LIPC expression. Undetectable levels of LIPC from 150 micrograms of bovine retinal extract prompted an immunoprecipitation (IP) experiment from 5 milligrams of bovine retinal lysate. Specificity of the 55 kDa LIPC band from the LIPC IP was validated using a negative control GFP IP. HSP90 was used to as an input loading control.
   (c) Real-time RT-PCR analysis of mouse retinal *Lipc* expression. Expression profiles of retinal *Lipc* were analyzed during postnatal development of C57BL/6 mice. P2, P14, P90 and P365 time points were selected to cover a broad window of development. Relative amounts of *Lipc* expression were compared to the expression of the *GAPDH* (glyceraldehyde-3-phosphate dehydrogenase) gene.
   (d) Agarose gel analysis of mouse *Lipc* expression. A 192 nucleotide-specific band was detected at P2, P14, P90 and P365. The *GAPDH* gene was used as a template control and could be detected as a 180 nucleotide band.

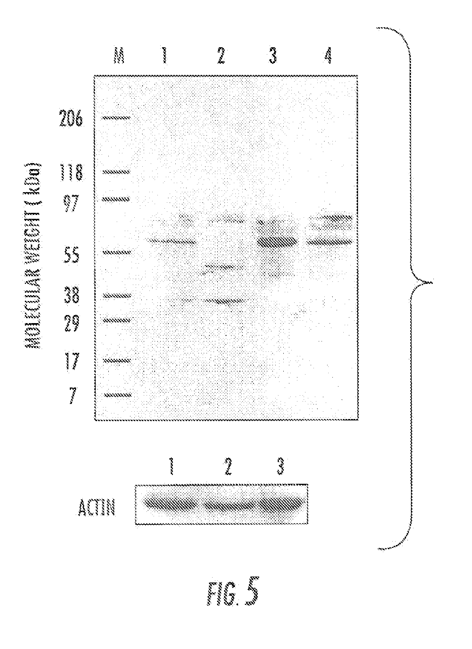

FIG 5. Immunoblot of hepatic lipase antibody (H-70, SC-21007) against human whole protein extracts. Lane 1, macular retina; Lane 2, peripheral retina; Lane 3, RPEchoroid; Lane 4, liver. Lanes 1-3 were normalized against actin.

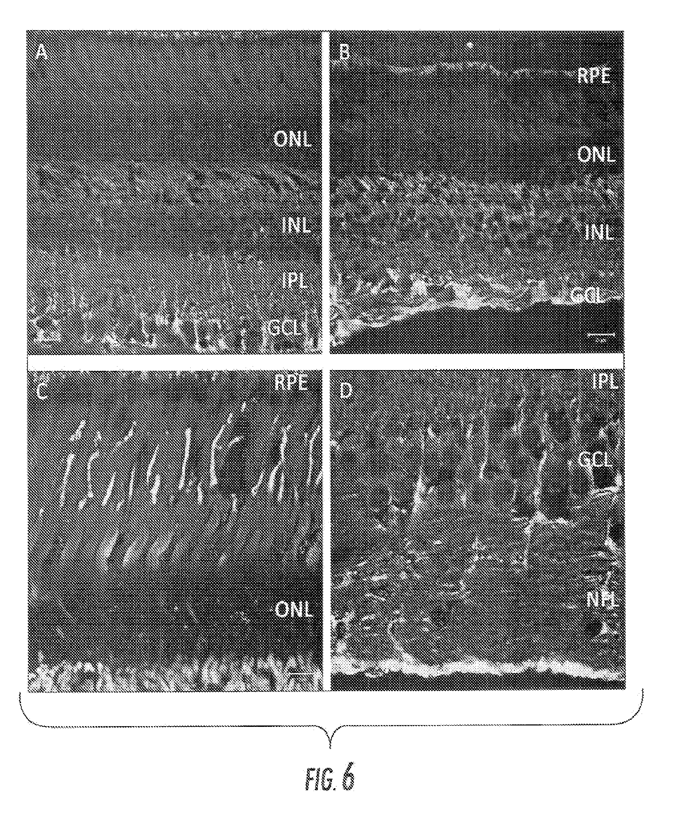

FIG 6. Distribution of hepatic lipase C (red) in central (A) and peripheral (B) monkey retina using a rabbit polyclonal antibody (Cat. #SC-21007) raised against amino acids 91-160 of human origin. This antibody labels all retina neurons, especially ganglion cells of central monkey retina, and does not label Mueller cells (identified by glutamine synthetase, green). High-magnification images of photoreceptors (C) and ganglion cells (D) to show immunoreactivity for anti-hepatic lipase C (red). Cones, defined by primate cone arrestin labeling (mAb 7G6, green in panel C), are weakly positive for hepatic lipase C. However, rods reveal strong punctate label (red) in the outer nuclear layer, as well as over inner and outer segments. In panel D, strong immunoreactivity for hepatic lipase C (red) observed in ganglion cell somata and axons of the nerve fiber layer (NFL) contrasts with that of a Mueller cell marker (glutamine synthetase, BD Transduction,green). Magnification bars = 20 µm (A,B) and 10 µm (C,D). Abbreviations: RPE, retina pigmented epithelium; ONL, outer nuclear layer; INL, inner nuclear layer; IPL, inner plexiform layer; GCL, ganglion cell layer.

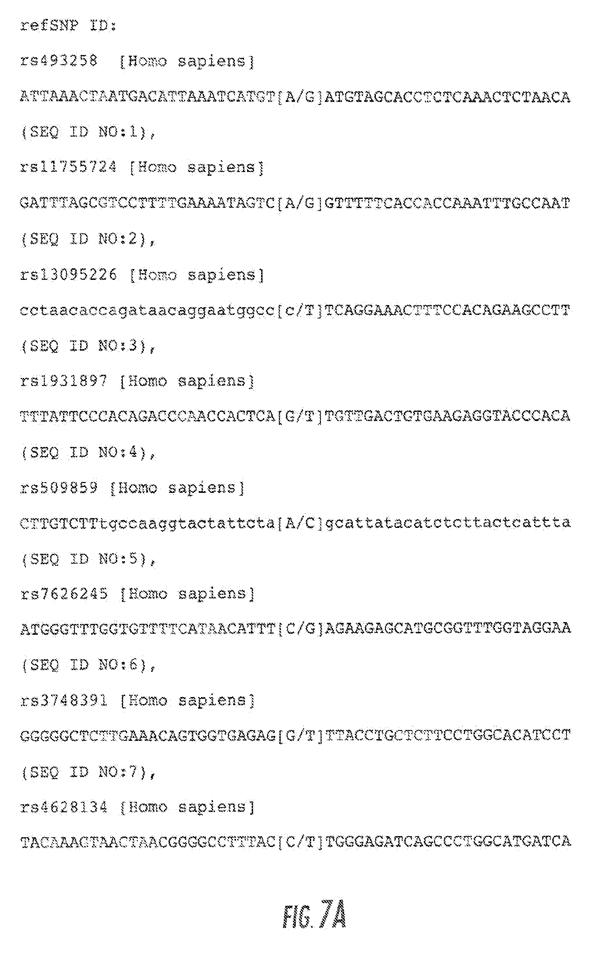

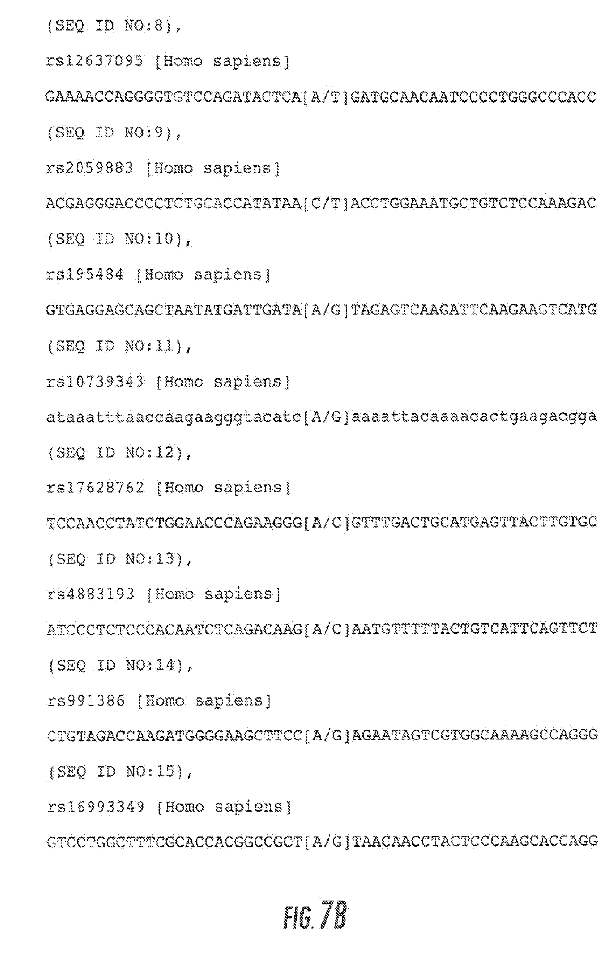

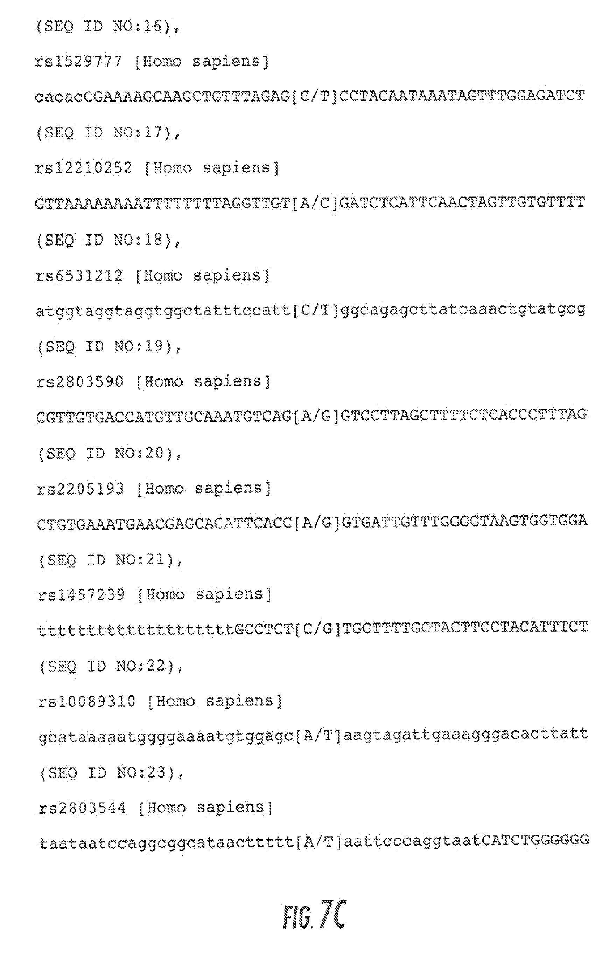

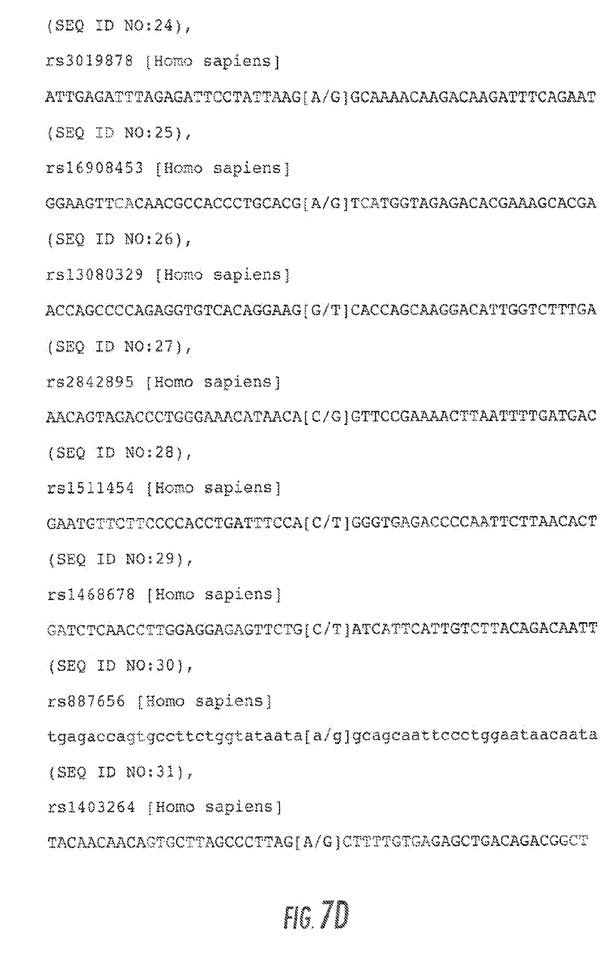

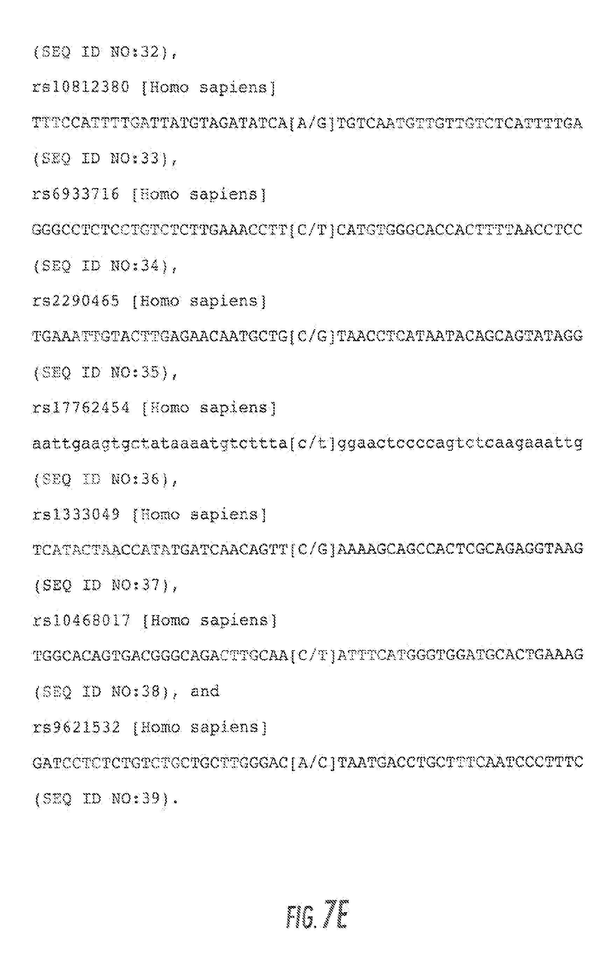

FIG. 7 are sequences showing alleles at polymorphic sites: rs493258 (SEQ ID NO:1), rs11755724 (SEQ ID NO:2), rs13095226 (SEQ ID NO:3), rs1931897 (SEQ ID NO:4), rs509859 (SEQ ID NO:5), rs7626245 (SEQ ID NO:6), rs3748391 (SEQ ID NO:7), rs4628134 (SEQ ID NO:8), rs12637095 (SEQ ID NO:9), rs2059883 (SEQ ID NO:10), rs195484 (SEQ ID NO:11), rs10739343 (SEQ ID NO:12), rs17628762 (SEQ ID NO:13), rs4883193 (SEQ ID NO:14), rs991386 (SEQ ID NO:15), rs16993349 (SEQ ID NO:16), rs1529777 (SEQ ID NO:17), rs12210252 (SEQ ID NO:18), rs6531212 (SEQ ID NO:19), rs2803590 (SEQ ID NO:20), rs2205193 (SEQ ID NO:21), rs1457239 (SEQ ID NO:22), rs10089310 (SEQ ID NO:23), rs2803544 (SEQ ID NO:24), rs3019878 (SEQ ID NO:25), rs16908453 (SEQ ID NO:26), rs13080329 (SEQ ID NO:27), rs2842895 (SEQ ID NO:28), rs1511454 (SEQ ID NO:29), rs1468678 (SEQ ID NO:30), rs887656 (SEQ ID NO:31), rs1403264 (SEQ ID NO:32), rs10812380 (SEQ ID NO:33), rs6933716 (SEQ ID NO:34), rs2290465 (SEQ ID NO:35), rs17762454 (SEQ ID NO:36), rs1333049 (SEQ ID NO:37), rs10468017 (SEQ ID NO:38), and rs9621532 (SEQ ID NO:39).

### Detailed Description

The present invention is directed to the discovery that particular alleles at polymorphic sites associated with genes coding for proteins associated with lipid metabolism, such as an associated SNP variant in the hepatic lipase gene (LIPC) in the high-density lipoprotein cholesterol (HDL-c) pathway, are useful as markers for AMD etiology and for determining susceptibility to AMD.

As used herein, "gene" is a term used to describe a genetic element that gives rise to expression products (*e.g.,* pre-mRNA, mRNA and polypeptides). A gene includes regulatory elements, exons and sequences that otherwise appear to have only structural features, *e.g.,* introns and untranslated regions.

The genetic markers are particular "alleles" at "polymorphic sites" associated with the hepatic lipase gene (LIPC). A nucleotide position at which more than one nucleotide can be present in a population (either a natural population or a synthetic population, *e.g.,* a library of synthetic molecules), is referred to herein as a "polymorphic site". Where a polymorphic site is a single nucleotide in length, the site is referred to as a single nucleotide polymorphism ("SNP"). If at a particular chromosomal location, for example, one member of a population has an adenine and another member of the population has a thymine at the same genomic position, then this position is a polymorphic site, and, more specifically, the polymorphic site is a SNP. Polymorphic sites can allow for differences in sequences based on substitutions, insertions or deletions. Each version of the sequence with respect to the polymorphic site is referred to herein as an "allele" of the polymorphic site. Thus, in the previous example, the SNP allows for both an adenine allele and a thymine allele.

A genetic marker is "associated" with a genetic element or phenotypic trait, for example, if the marker is co-present with the genetic element or phenotypic trait at a frequency that is higher than would be predicted by random assortment of alleles (based on the allele frequencies of the particular population). Association also indicates physical association, *e.g.,* proximity in the genome or presence in a haplotype block, of a marker and a genetic element.

A reference sequence is typically referred to for a particular genetic element, *e.g.,* a gene. Alleles that differ from the reference are referred to as "variant" alleles. The reference sequence, often chosen as the most frequently occurring allele or as the allele conferring a typical phenotype, is referred to as the "wild-type" allele.

Some variant alleles can include changes that affect a polypeptide, *e.g.,* the polypeptide encoded by a complement pathway gene. These sequence differences, when compared to a reference nucleotide sequence, can include the insertion or deletion of a single nucleotide, or of more than one nucleotide, resulting in a frame shift; the change of at least one nucleotide, resulting in a change in the encoded amino acid; the change of at least one nucleotide, resulting in the generation of a premature stop codon; the deletion of several nucleotides, resulting in a deletion of one or more amino acids encoded by the nucleotides; the insertion of one or several nucleotides, such as by unequal recombination or gene conversion, resulting in an interruption of the coding sequence of a reading frame; duplication of all or a part of a sequence; transposition; or a rearrangement of a nucleotide sequence.

Alternatively, a polymorphism associated with AMD or a susceptibility to AMD can be a synonymous change in one or more nucleotides (*i.e.,* a change that does not result in a change to a codon of a complement pathway gene). Such a polymorphism can, for example, alter splice sites, affect the stability or transport of mRNA, or otherwise affect the transcription or translation of the polypeptide. The polypeptide encoded by the reference nucleotide sequence is the "reference" polypeptide with a particular reference amino acid sequence, and polypeptides encoded by variant alleles are referred to as "variant" polypeptides with variant amino acid sequences.

Haplotypes are a combination of genetic markers, e.g., particular alleles at polymorphic sites. The haplotypes described herein are associated with AMD and/or a susceptibility to AMD. Detection of the presence or absence of the haplotypes herein, therefore is indicative of AMD, a susceptibility to AMD or a lack thereof. The haplotypes described herein are a combination of genetic markers, *e.g.,* SNPs and microsatellites. Detecting haplotypes, therefore, can be accomplished by methods known in the art for detecting sequences at polymorphic sites.

The haplotypes and markers disclosed herein are in "linkage disequilibrium" (LD) with hepatic lipase gene (LIPC) in the high-density lipoprotein cholesterol (HDL-c) pathway, and likewise, AMD and high-density lipoprotein cholesterol-associated phenotypes. "Linkage" refers to a higher than expected statistical association of genotypes and/or phenotypes with each other. LD refers to a non-random assortment of two genetic elements. If a particular genetic element (*e.g.,* an allele at a polymorphic site), for example, occurs in a population at a frequency of 0.25 and another occurs at a frequency of 0.25, then the predicted occurrence of a person's having both elements is 0.125, assuming a random distribution of the elements. If, however, it is discovered that the two elements occur together at a frequency higher than 0.125, then the elements are said to be in LD since they tend to be inherited together at a higher frequency than what their independent allele frequencies would predict. Roughly speaking, LD is generally correlated with the frequency of recombination events between the two elements. Allele frequencies can be determined in a population, for example, by genotyping individuals in a population and determining the occurrence of each allele in the population. For populations of diploid individuals, *e.g.,* human populations, individuals will typically have two alleles for each genetic element (*e.g.,* a marker or gene).

The invention is also directed to markers identified in a "haplotype block" or "LD block". These blocks are defined either by their physical proximity to a genetic element, *e.g.,* a lipoprotein pathway gene, or by their "genetic distance" from the element. Markers and haplotypes identified in these blocks, because of their association with AMD and the lipoprotein metabolic pathways, are encompassed by the invention. One of skill in the art will appreciate regions of chromosomes that recombine infrequently and regions of chromosomes that are "hotspots", *e.g.,* exhibiting frequent recombination events, are descriptive of LD blocks. Regions of infrequent recombination events bounded by hotspots will form a block that will be maintained during cell division. Thus, identification of a marker associated with a phenotype, wherein the marker is contained within an LD block, identifies the block as associated with the phenotype. Any marker identified within the block can therefore be used to indicate the phenotype.

Additional markers that are in LD with the markers of the invention or haplotypes are referred to herein as "surrogate" markers. Such a surrogate is a marker for another marker or another surrogate marker. Surrogate markers are themselves markers and are indicative of the presence of another marker, which is in turn indicative of either another marker or an associated phenotype.

Susceptibility for developing AMD includes an asymptomatic patient showing increased risk to develop AMD, and a patient having AMD indicating a progression toward more advanced forms of AMD. Susceptibility for not developing AMD includes an asymptomatic patient, wherein the presence of the wild type allele indicating a lack of predisposition to AMD.

Several candidate genes have been screened negatively for association with AMD. These include TIMP3 (Tissue Inhibitor of MetalloProteinases-3), IMPG2, the gene encoding the retinal interphotoreceptor matrix (IPM) proteoglycan IPM 200, VMD2 (the bestrophin gene), ELOVL4 (elongation of very long chain fatty acids), RDS (peripherin), EFEMP1 (EGF-containing fibulin-like extracellular matrix), BMD (bestrophin). One gene has been shown to have variations in the coding regions in patients with AMD, GPR75 (a G protein coupled receptor gene). Others have shown a possible association with the disease in at least one study-PON1 the (paraoxonase gene); SOD2 (manganese superoxide dismutase; APOE (apolipoprotein E), in which the ε4 allele has been found to be associated with the disease in some studies and not associated in others; and CST3 (cystatin C), where one study has suggested an increased susceptibility for ARMD in CST3 B/B homozygotes. There are conflicting reports regarding the role of the ABCR (ABCA4) gene with regard to AMD.

### Diagnostic Gene Array

In one aspect, the invention comprises an array of gene fragments, particularly including those SNPs given as SEQ ID NOS:1-39, and probes for detecting the allele at the SNPs of SEQ ID NOS:1-39. Polynucleotide arrays provide a high throughput technique that can assay a large number of polynucleotide sequences in a single sample. This technology can be used, for example, as a diagnostic tool to assess the risk potential of developing AMD using the SNPs and probes of the invention. Polynucleotide arrays (for example, DNA or RNA arrays), include regions of usually different sequence polynucleotides arranged in a predetermined configuration on a substrate, at defined *x* and *y* coordinates. These regions (sometimes referenced as "features") are positioned at respective locations ("addresses") on the substrate. The arrays, when exposed to a sample, will exhibit an observed binding pattern. This binding pattern can be detected upon interrogating the array. For example all polynucleotide targets (for example, DNA) in the sample can be labeled with a suitable label (such as a fluorescent compound), and the fluorescence pattern on the array accurately observed following exposure to the sample. Assuming that the different sequence polynucleotides were correctly deposited in accordance with the predetermined configuration, then the observed binding pattern will be indicative of the presence and/or concentration of one or more polynucleotide components of the sample.

Arrays can be fabricated by depositing previously obtained biopolymers onto a substrate, or by *in situ* synthesis methods. The substrate can be any supporting material to which polynucleotide probes can be attached, including but not limited to glass, nitrocellulose, silicon, and nylon. Polynucleotides can be bound to the substrate by either covalent bonds or by non-specific interactions, such as hydrophobic interactions. The *in situ* fabrication methods include those described in U.S. Pat. No. 5,449,754 for synthesizing peptide arrays, and in U.S. Pat. No. 6,180,351 and WO 98/41531 and the references cited therein for synthesizing polynucleotide arrays. Further details of fabricating biopolymer arrays are described in U.S. Pat. No. 6,242,266; U.S. Pat. No. 6,232,072; U.S. Pat. No. 6,180,351; U.S. Pat. No. 6,171,797; EP No. 0 799 897; PCT No. WO 97/29212; PCT No. WO 97/27317; EP No. 0 785 280; PCT No. WO 97/02357; U.S. Pat. Nos. 5,593,839; 5,578,832; EP No. 0 728 520; U.S. Pat. No. 5,599,695; EP No. 0 721 016; U.S. Pat. No. 5,556,752; PCT No. WO 95/22058; and U.S. Pat. No. 5,631,734. Other techniques for fabricating biopolymer arrays include known light directed synthesis techniques. Commercially available polynucleotide arrays, such as Affymetrix GeneChip™, can also be used. Use of the GeneChip™, to detect gene expression is described, for example, in Lockhart et al., Nat. Biotechnol., 14:1675, 1996; Chee et al., Science, 274:610, 1996; Hacia et al., Nat. Gen., 14:441, 1996; and Kozal et al., Nat. Med., 2:753, 1996. Other types of arrays are known in the art, and are sufficient for developing an AMD diagnostic array of the present invention.

To create the arrays, single-stranded polynucleotide probes can be spotted onto a substrate in a two-dimensional matrix or array. Each single-stranded polynucleotide probe can comprise at least 6, 7, 8, 9, 10, 11, 12, 13, 14, 15, 16, 17, 18, 19, 20, 25, or 30 or more contiguous nucleotides selected from the nucleotide sequences shown in SEQ ID NO:1-39, or the complement thereof. Preferred arrays comprise at least one single-stranded polynucleotide probe comprising at least 6, 7, 8, 9, 10, 11, 12, 13, 14, 15, 16, 17, 18, 19, 20, 25, or 30 or more contiguous nucleotides selected from the nucleotide sequences shown in SEQ ID NO:1-39, or the complement thereof.

Tissue samples from a subject can be treated to form single-stranded polynucleotides, for example by heating or by chemical denaturation, as is known in the art. The single-stranded polynucleotides in the tissue sample can then be labeled and hybridized to the polynucleotide probes on the array. Detectable labels that can be used include but are not limited to radiolabels, biotinylated labels, fluorophors, and chemiluminescent labels. Double stranded polynucleotides, comprising the labeled sample polynucleotides bound to polynucleotide probes, can be detected once the unbound portion of the sample is washed away. Detection can be visual or with computer assistance. Preferably, after the array has been exposed to a sample, the array is read with a reading apparatus (such as an array "scanner") that detects the signals (such as a fluorescence pattern) from the array features. Such a reader preferably would have a very fine resolution (for example, in the range of five to twenty microns) for an array having closely spaced features.

The signal image resulting from reading the array can then be digitally processed to evaluate which regions (pixels) of read data belong to a given feature as well as to calculate the total signal strength associated with each of the features. The foregoing steps, separately or collectively, are referred to as "feature extraction" (U.S. Pat No. 7,206,438). Using any of the feature extraction techniques so described, detection of hybridization of a patient derived polynucleotide sample with one of the AMD markers on the array given as SEQ ID NO:1-39 identifies that subject as having or not having a genetic risk factor for AMD, as described above.

### System for Analyzing Patient Data

In another aspect, the invention provides a system for compiling and processing patient data, and presenting a risk profile for developing AMD. A computer aided medical data exchange system is preferred. The system is designed to provide high-quality medical care to a patient by facilitating the management of data available to care providers. The care providers will typically include physicians, surgeons, nurses, clinicians, various specialists, and so forth. It should be noted, however, that while general reference is made to a clinician in the present context, the care providers may also include clerical staff, insurance companies, teachers and students, and so forth. The system provides an interface, which allows the clinicians to exchange data with a data processing system. The data processing system is linked to an integrated knowledge base and a database.

The database may be software-based, and includes data access tools for drawing information from the various resources as described below, or coordinating or translating the access of such information. In general, the database will unify raw data into a useable form. Any suitable form may be employed, and multiple forms may be employed, where desired, including hypertext markup language (HTML) extended markup language (XML), Digital Imaging and Communications in Medicine (DICOM), Health Level Seven™ (HL7), and so forth. In the present context, the integrated knowledge base is considered to include any and all types of available medical data that can be processed by the data processing system and made available to the clinicians for providing the desired medical care. In general, data within the resources and knowledge base are digitized and stored to make the data available for extraction and analysis by the database and the data processing system. Even where more conventional data gathering resources are employed, the data is placed in a form that permits it to be identified and manipulated in the various types of analyses performed by the data processing system.

The integrated knowledge base is intended to include one or more repositories of medical-related data in a broad sense, as well as interfaces and translators between the repositories, and processing capabilities for carrying out desired operations on the data, including analysis, diagnosis, reporting, display and other functions. The data itself may relate to patient-specific characteristics as well as to non-patient specific information, as for classes of persons, machines, systems and so forth. Moreover, the repositories may include devoted systems for storing the data, or memory devices that are part of disparate systems, such as imaging systems. As noted above, the repositories and processing resources making up the integrated knowledge base may be expandable and may be physically resident at any number of locations, typically linked by dedicated or open network links. Furthermore, the data contained in the integrated knowledge base may include both clinical data (*e.g.,* data relating specifically to a patient condition) and non-clinical data. Examples of preferred clinical data include patient medical histories, patient serum, plasma, and/or other biomarkers such as blood levels of certain other nutrients, fats, female and male hormones, etc., and cellular antioxidant levels, and the identification of past or current environmental, lifestyle and other factors that predispose a patient to develop AMD. These include but are not limited to various risk factors such as obesity, smoking, vitamin and dietary supplement intake, use of alcohol or drugs, poor diet and a sedentary lifestyle. Non-clinical data may include more general information about the patient, such as residential address, data relating to an insurance carrier, and names and addresses or phone numbers of significant or recent practitioners who have seen or cared for the patient, including primary care physicians, specialists, and so forth.

The flow of information can include a wide range of types and vehicles for information exchange. In general, the patient can interface with clinicians through conventional clinical visits, as well as remotely by telephone, electronic mail, forms, and so forth. The patient can also interact with elements of the resources via a range of patient data acquisition interfaces that can include conventional patient history forms, interfaces for imaging systems, systems for collecting and analyzing tissue samples, body fluids, and so forth. Interaction between the clinicians and the interface can take any suitable form, depending upon the nature of the interface. Thus, the clinicians can interact with the data processing system through conventional input devices such as keyboards, computer mice, touch screens, portable or remote input and reporting devices. The links between the interface, data processing system, the knowledge base, the database and the resources typically include computer data exchange interconnections, network connections, local area networks, wide area networks, dedicated networks, virtual private network, and so forth.

In general, the resources can be patient-specific or patient-related, that is, collected from direct access either physically or remotely (*e.g.,* via computer link) from a patient. The resource data can also be population-specific so as to permit analysis of specific patient risks and conditions based upon comparisons to known population characteristics. It should be noted that the resources can generally be thought of as processes for generating data. While many of the systems and resources will themselves contain data, these resources are controllable and can be prescribed to the extent that they can be used to generate data as needed for appropriate treatment of the patient. Exemplary controllable and prescribable resources include, for example, a variety of data collection systems designed to detect physiological parameters of patients based upon sensed signals. Such electrical resources can include, for example, electroencephalography resources (EEG), electrocardiography resources (ECG), electromyography resources (EMG), electrical impedance tomography resources (EIT), nerve conduction test resources, electronystagmography resources (ENG), and combinations of such resources. Various imaging resources can be controlled and prescribed as indicated at reference numeral. A number of modalities of such resources are currently available, such as, for example, X-ray imaging systems, magnetic resonance (MR) imaging systems, computed tomography (CT) imaging systems, positron emission tomography (PET) systems, fluorography systems, sonography systems, infrared imaging systems, nuclear imaging systems, thermoacoustic systems, and so forth. Imaging systems can draw information from other imaging systems, electrical resources can interface with imaging systems for direct exchange of information (such as for timing or coordination of image data generation, and so forth).

In addition to such electrical and highly automated systems, various resources of a clinical and laboratory nature can be accessible. Such resources may include blood, urine, saliva and other fluid analysis resources, including gastrointestinal, reproductive, urological, nephrological (kidney function), and cerebrospinal fluid analysis system. Such resources can further include polymerase (PCR) chain reaction analysis systems, genetic marker analysis systems, radioimmunoassay systems, chromatography and similar chemical analysis systems, receptor assay systems and combinations of such systems. Histologic resources, somewhat similarly, can be included, such as tissue analysis systems, cytology and tissue typing systems and so forth. Other histologic resources can include immunocytochemistry and histopathological analysis systems. Similarly, electron and other microscopy systems, *in situ* hybridization systems, and so forth can constitute the exemplary histologic resources. Pharmacokinetic resources can include such systems as therapeutic drug monitoring systems, receptor characterization and measurement systems, and so forth. Again, while such data exchange can be thought of passing through the data processing system, direct exchange between the various resources can also be implemented.

Use of the present system involves a clinician obtaining a patient sample, and evaluation of the presence of a genetic marker in that patient indicating a predisposition (or not) for AMD, such as SEQ ID NO:1-39, alone or in combination with other known risk factors. The clinician or their assistant also obtains appropriate clinical and non-clinical patient information, and inputs it into the system. The system then compiles and processes the data, and provides output information that includes a risk profile for the patient, of developing AMD.

The present invention thus provides for certain polynucleotide sequences that have been correlated to AMD. These polynucleotides are useful as diagnostics, and are preferably used to fabricate an array, useful for screening patient samples. The array, in a currently most preferred embodiment, is used as part of a laboratory information management system, to store and process additional patient information in addition to the patient's genomic profile. As described herein, the system provides an assessment of the patient's risk for developing AMD, risk for disease progression, and likelihood of disease prevention based on patient controllable factors.

### EXAMPLES

### Example 1.

### Discovery of genetic variants associated with AMD:

Age-related macular degeneration (AMD), the leading cause of late onset blindness, arises from retinal damage associated with accumulation of drusen and subsequent atrophy or neovascularization that leads to loss of central vision. The results of a genome-wide association study (GWAS) of 979 advanced AMD cases and 1709 controls using the Affymetrix 6.0 platform with replication in seven additional cohorts (totaling 4337 unrelated cases and unrelated 2077 controls) are presented. The first comprehensive analysis of copy number variations and polymorphisms for AMD is also presented. These data implicated an associated variant in the hepatic lipase gene (LIPC) in the high-density lipoprotein cholesterol (HDL-c) pathway (discovery P = 4.53e-05, combined P =2.2e-09 for rs493258). This association was independently corroborated by the Michigan/Penn/Mayo (MPM) GWAS. The locus near tissue inhibitor of metalloproteinase 3 (TIMP3) was confirmed by our replication cohort for re9621532 with P = 3.71e-09. The LIPC association was strongest for a functional promoter variant, rs10468017, with P = 1.34e-08 (independent of MPM data), that influences LIPC expression and serum HDL levels, with a protective effect of the minor Y allele (HDL increasing) for advanced wet and dry AMD. Weaker associations with other HDL loci (ABCA1, P = 9.73e-04; CETP, P = 1.41e-03; FADS1-3, P = 2.69e-02) were also observed. Based upon a lack of consistent association between HDL increasing alleles and AMD risk, the LIPC association may not be the result of an effect on HDL levels, but could represent a pleiotropic effect of the same functional component. This genetic locus implicates a different biologic pathway than previously reported and provides a new avenue for possible prevention and treatment of AMD.

Age-related macular degeneration (AMD) is a common, late-onset disorder that is modified by covariates including smoking and BMI, and has a 3-6 fold higher recurrence ratio in siblings than in the general population. The burden of AMD is clinically significant, causes visual loss, and reduces quality of life. Among individuals age 75 or older, approximately one in four have some sign of this disease, while about one in 15 have the advanced form with visual loss. Small genome-wide association studies (GWAS), candidate gene screening, and focused examination of regions identified in linkage studies revealed significant associations between AMD and common genetic variations at CFH loci on chromosome 1 and the ARMS2/HTRA1 region on chromosome 10.

The discovery of the *CFH* association revealed a role for the complement pathway in disease pathogenesis. Further genetic focus on this pathway has revealed three additional risk factors at the *CFB*/*C2* locus on chromosome 6, *C3* on chromosome 19 and *CFI* on chromosome 4, indicating the critical role of this pathway. Together with variants in the *ARMS2*/*HTRA1* region, these multiple loci have been estimated to explain roughly half of the heritability of AMD, and combined with demographic and environmental factors have high potential predictive power. To date, however, no large-scale genome-wide association studies have been undertaken to attempt to explain the remaining heritability of AMD, and to identify susceptibility loci outside of the complement system and the chromosome 10 region that may contribute to AMD pathogenesis. Described herein is such a study involving 979 cases of advanced AMD in the discovery phase with multiple stages of replication. Samples were genotyped on the Affymetrix 6.0 platform which contains probes for 906,000 SNPs and an additional 946,000 SNP-invariant probes to enhance copy number variation (CNV) analysis and captures 82% of the variation at an r² ≥ 0.8 for Europeans in the 3.1 million SNPs of HapMap phase 2. These studies have uncovered several new AMD susceptibility loci. Intriguingly, the most significant, replicated association is a functional variation in LIPC (hepatic lipase), a gene involved in triglyceride hydrolysis and high density lipoprotein (HDL) function, thus revealing a novel pathway involved in AMD pathogenesis.

### RESULTS

**Case and Control Sample Development.** The initial study consisted of 1,057 unrelated cases with geographic atrophy or neovascular AMD, and 558 unrelated controls without AMD who were phenotyped based on clinical examination and ocular photography, and identified from studies of genetic-epidemiology of macular degeneration at Tufts Medical Center. The AMD grade in the worst eye was used in the analyses. All individuals were Caucasian from European ancestry (further details about the original and replication study populations can be found in METHODS and Table 1).

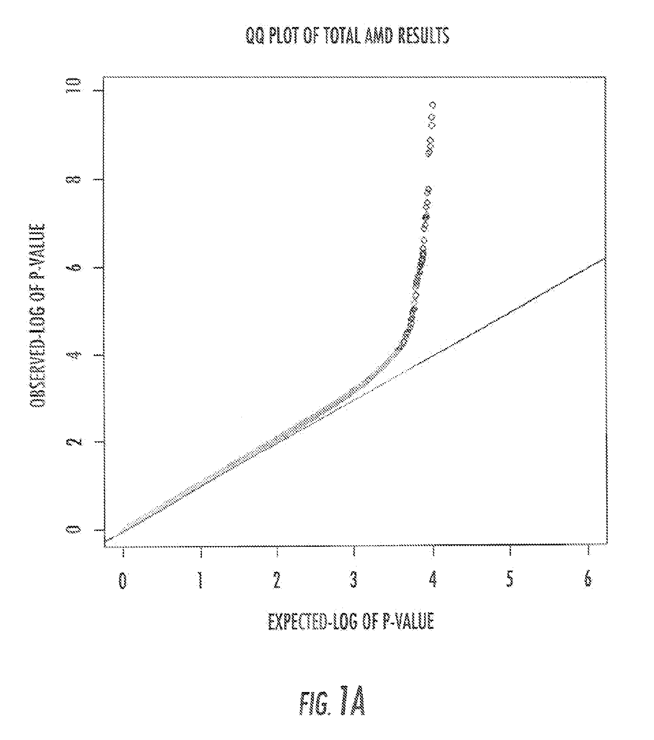

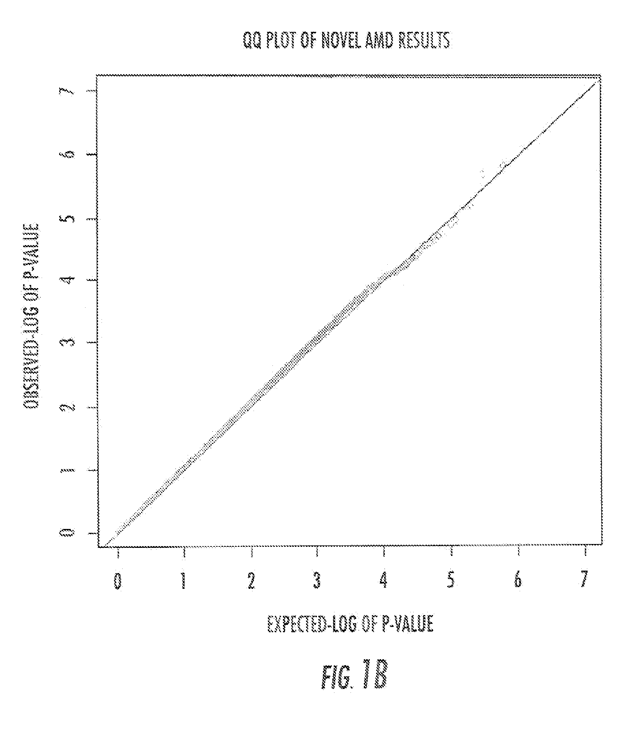

To enhance the power of this study, unrelated control resources that were genotyped on the same platform in the same lab were included, and additional stringent quality control to ensure the technical and population compatibility of these datasets was conducted (METHODS and Table 2). The final genotyped sample consisted of 979 cases and 1,709 controls. Using a logistic regression analysis including population structure covariates, genomic control inflation factors were comparable between the initial, similarly ascertained sample and the expanded sample, suggesting that potential population differences have been controlled appropriately (979 cases to 536 controls lamba = 1.051; 979 cases to 1,709 controls lambda = 1.036). Because these additional controls were unscreened for AMD status and may include individuals who have or might later develop AMD, their impact on established associations was determined. The most compelling previously reported associated regions in AMD: *CFH* on chromosome 1, *CFI* on chromosome *4, BF*/*C2* on chromosome 6, *ARMS2*/*HTRA1* on chromosome 10, and *C3* on chromosome 19 were examined. 159 SNPs that were in LD with the most positively associated variant reported in the literature were examined. Of these, 137 showed an improvement in the χ², with the addition of these controls. The average ratio of the initial study's cleaned χ² to final study's cleaned χ² was 1.82- nearly identical to the expected improvement in χ² based on theoretical power calculations of 1.84. As predicted, the addition of a significant number of unselected controls increased the power of this study substantially.
**Genome-Wide Association Discovery Phase.** Using a case-control analysis as implemented in PLINK, no SNPs in regions not already reported as being associated with AMD achieved genome-wide significance of 5 × 10⁻⁸ as defined by Pe'er *et al.* The genome-wide association findings from the final dataset were plotted in Quantile-Quantile (QQ) plots. The top end of the QQ plot (shown in Figure 1A) is dominated by the strong associations of previously reported SNPs. As these associations are well-validated, they were removed to more fully examine the remaining distribution of association results (shown in Figure 1B). The regions of previously reported association are near CFH (rs572515 proxy for rs1061170 with r²=0.654 and with P<10⁻⁵⁵, rs10737680 proxy for CFH rs1410996 with r²=1 and with P<10⁻⁴⁷), near CFI (rs7690921 proxy for rs10033900 with r²=0.403 and with P<10⁻⁴), near CFB/C2 (rs522162 proxy for rs641153 with r²=1 and with P<10⁻⁶), and the ARMS2/HTRA1 locus (rs10490924 with P<10⁻⁵⁹) (Table 3). There were no adequate proxies for the C3 locus. Several SNPs of interest in regions without previously reported association with P-values between 10⁻⁴ to 10⁻⁶ were identified in the discovery scan (Table 7a and Tables 8), including rs493258 (LIPC) with P = 4.5 × 10⁻⁵, as discussed in more detail below.
**Replication Phases.** To evaluate the top results from novel regions identified by the scan, several stages of replication analysis were performed. For all SNPs with p < 10⁻³ in the genome-wide association scan, results were obtained from the Michigan, Penn, and Mayo scan, selecting only their advanced cases versus controls, and combined the study results as equally weighted-Z scores given the similar sample sizes. From this combined analysis, SNPs with p < 10⁻⁴ or higher in our independent local replication sample of advanced cases and controls from Tufts University School of Medicine and Massachusetts General Hospital (Tufts/MGH) were genotyped, who were unrelated to the individuals in our original scan. Not all SNPs could be imputed perfectly in the Michigan scan, given the different sizes and types of genotyping platforms used (Affymetrix 6.0 with 906,000 SNPS and Illumina with 320,000 SNPS). Therefore, a subset of strongly associated SNPs from the scan alone were selected to be genotyped in the local replication sample. After these steps, a subset of promising SNPs were distributed to collaborators at Johns Hopkins University (JHU) and Columbia University (COL), University of Utah (UT), Hopital Intercommunal de Creteil (FR-CRET), and Washington University (WASH-U), for replication in independent samples. The most significantly associated SNPs from our overall analyses are presented in Tables 8 and 9. No evidence for other recently reported candidate gene associations to AMD were found including TLR3, SERPING1, LRP6, PEDF, VEGF, TLR4, CX3CR1, ELOVL4, PON1, or SOD2 (Table 4) in the discovery samples. A tally of these P-values for the discovery and local replication stages are presented in Table 5.

Notably, while all previously reported associations in the complement pathway and the strong *ARMS2*/*HTRA1* association are clearly observed (Table 7b), no supporting evidence was found for other recently reported candidate associations (Table 4). As this study, in combination with the accompanying Michigan scan, which similarly finds no evidence of association at these loci, constitute a much larger sample than the original reports of proposed association, the conclusions is reached that many of these reports constitute false positive findings.
**Results of Combined Scan and Replication Analysis.** The SNP on chromosome 15, rs493258 showed significant association with P = 1.61 × 10⁻⁸. This SNP is located 35 kb upstream of the hepatic lipase (LIPC) gene on chromosome 15q22 and is in LD (r²=0.33; D' = 0.847) with the previously described functional variant (rs10468017) reported to influence LIPC expression and serum HDL levels. To evaluate whether this new association represented an effect of that same functional variant, we genotyped rs10468017 in all replication samples except MPM (Table 9). The resultant P-value was 1.34 x10⁻⁸. This SNP indeed demonstrated stronger association than rs493258 (replication chi-sq: rs493258=6.7, rs10468017=16.1; minor allele T, frequency among cases = 25.8%, and frequency among controls = 30.0%). Conditional logistic regression analyses indicated that rs493258 is not associated conditional on rs10468017, yet rs10468017 is still associated (p<.001) conditional on association at rs493258. Therefore, it is concluded that this AMD association represents the same functional variant identified in HDL studies. The estimated odds ratio (OR) for rs10468017 from the combined discovery plus replication data is 0.82 (95% confidence interval 0.76-0.88), comparing the TT to the CC genotype, demonstrating that the minor allele T, previously associated with reduced LIPC expression and higher HDL, is also associated with reduction in risk of AMD. This SNP, like those in the complement pathway, is observed to have a consistent odds ratio among geographic atrophy ("advanced dry", OR = 0.73) and neovascular ("wet", OR = 0.77) AMD when each disease subtype is compared to controls. No significant gene-gene interactions were found between this SNP and seven previously established genetic loci when evaluating their effect on risk of advanced AMD. For rs10468017, there is modest evidence for heterogeneity under the Breslow-Day test for the replication samples shown in Table 9 (P = 0.020), with the Utah (Breslow-Day P = 2.7 x 10⁻³) sample lacking association, and the French (Breslow-Day P = 0.040) sample having higher than expected association, being the primary sources of heterogeneity to this SNP (Table 6). If these two samples were removed the result of association is stronger (P = 6.45 x 10⁻⁹) than with them included in the analysis (P = 1.34 x 10⁻⁸). Rather than discounting these samples based upon modest heterogeneity, particularly given the fact that both samples show association to complement loci, such heterogeneity could arise from yet to be discovered genotype or phenotype correlations and ascertainment differences between these samples that may prove informative to investigate.

The top SNPs for advanced wet and dry AMD phenotypes separately compared to controls in the overall scan data were examined. With the exception of the ARMS2 locus on chromosome 10 which is more strongly related to progression to neovascular AMD (OR = 1.38, P = 0.0034 comparing wet cases to dry cases), none of the other top SNPs advanced for replication showed significant differences between the two subtypes of advanced AMD and controls. Moreover, when comparing these two types versus controls across the genome, no associations with p < 10⁻⁶ were observed, and no excess of SNPs with p < 10⁻⁴ beyond the number expected by chance was observed, suggesting any differences if present are not likely to be large. This scan did not have sufficient statistical power to conclusively highlight weak or moderate subtype specific SNP associations between these two advanced forms of AMD.

Given the association of AMD to a SNP known to significantly influence serum HDL levels, the possibility that HDL might constitute a risk factor for AMD was considered. To evaluate this, a list of previously reported HDL associations was utilized. Among the best proxy SNPs for six other HDL associated variants, none were even nominally associated to AMD and no consistent trend between HDL lowering alleles and AMD risk was observed (Table 10). When these discovery results were combined with the replication samples (independent of MPM data), the meta P was 1.41 x 10⁻³ for the cholesterylester protein (CETP) SNP, rs3764261. However, the HDL increasing allele, A, was in the direction of increased risk for AMD, opposite to the protective effect seen with the functional HDL increasing LIPC variant. A variant, rs12678919, in lipoprotein lipase precursor (LPL) with meta P = 0.07, as well as rs1883025 in ATP-binding cassette, sub-family A member 1 (ABCA1) with meta P = 9.73 x 10⁻⁴, were also not strongly associated with AMD, and show discordant effects between the HDL increasing allele and protective or risk effects for AMD. It was concluded that the association found between advanced AMD and LIPC may not represent phenotypic correlation to, or causal effect of serum HDL, but rather could indicate a shared underlying biological mechanism involving the cholesterol pathway.
**Other loci:** In the discovery scan, the SNP rs9621532, in the synapsin III gene, a little more than 100kb upstream of the tissue inhibitor of metalloproteinase 3 (TIMP3) locus, the main finding of the MPM scan, had P = 0.045. Combined with the replication cohorts, (not including the MPM GWAS data), this SNP showed P = 3.71 x 10⁻⁹ in the direction of a protective effect for the minor C allele (OR=0.62). Of note, the SNP rs9621532 was not on the list of top SNPs, and the SNPs at the LIPC locus were not on the list of top SNPs from the MPM scan. TIMP3 has been found to be a matrix-bound angiogenesis inhibitor and mutations in the gene itself have been shown to induce abnormal neovascularization. Our study of the TIMP3 locus and AMD in 1997, inspired by the association of this gene to Sorsby's fundus dystrophy which has similar phenotypic features to advanced AMD, did not find evidence of association or linkage between AMD and TIMP3 among 38 families. However, as it was stated "the possibiliby that a subset of cases could be caused by the TIMP 3 locus could not be ruled out" by this relatively small study (DeLaPaz et al., 1997, Invest Ophthalmol Vis Sci, Vol 38, pgs 1060-1065).

Several regions in the genome-wide scan other than LIPC continue to show consistent association after several rounds of replication, although not at levels of genome-wide significance (Table 8). The SNP with the next highest final combined P-value is rs1755724 on chromosome 6, showing protective association with the minor A allele to AMD with P = 9.88 x 10-7 and an OR = 0.87. It is an intronic SNP in the locus for the RAS responsive element binding protein 1 isoform (RREB1). This gene encodes a transcription factor that binds specifically to the RAS-responsive elements of gene promoters. The SNP rs13095226 on chromosome 3, P = 2.50 x 10-6 and OR = 1.24 for the C allele, is an intronic SNP in the COL8A1 gene. COL8A1 encodes one of the two alpha chains of type VIII collagen. The gene product is a short chain collagen and a major component of the multiple basement membranes in the eye including Bruch's membrane and the choroidal stroma. Bruch's membrane is located directly below the retinal pigment epithelium and plays a central role in the pathogenesis of AMD.
**Copy Number Variations.** In addition to SNP variation, the expanded capabilities of the Affymetrix 6.0 platform was used in this study to assess the role of common and rare copy-number variations (CNVs) in AMD. Advanced AMD cases and controls from the phenotyped sample only and excluded MIGEN controls were examined. CANARY was first used to evaluate 554 common segregating copy-number polymorphisms (CNPs) and observed no CNPs with p < 0.001 with the exception of the previously reported deletion variant at CFHR1 which demonstrated strong association (P = 1 x 10-19). This observation is explained by other previously reported CFH variants associated with AMD, in particular the intronic SNP rs1410996, and does not constitute an independent association. Birdseye was utilized to examine the potential role of rare CNVs. After stringent quality control, a set of 150 rare deletions and 278 rare duplications greater than 100 kb was observed. However, these events were not enriched genome-wide among cases compared with controls, nor were there specific regions showing association of a rare CNV (Fisher's exact test p < 0.01). No evidence was found for any rare CNVs disrupting genes at the six confirmed associated loci. Overall, therefore, no strong evidence was found for novel association to CNPs or rare CNVs in AMD.

### Immunoprecipitation Real-Time PCR, and Immunohistochemistry of LIPC.

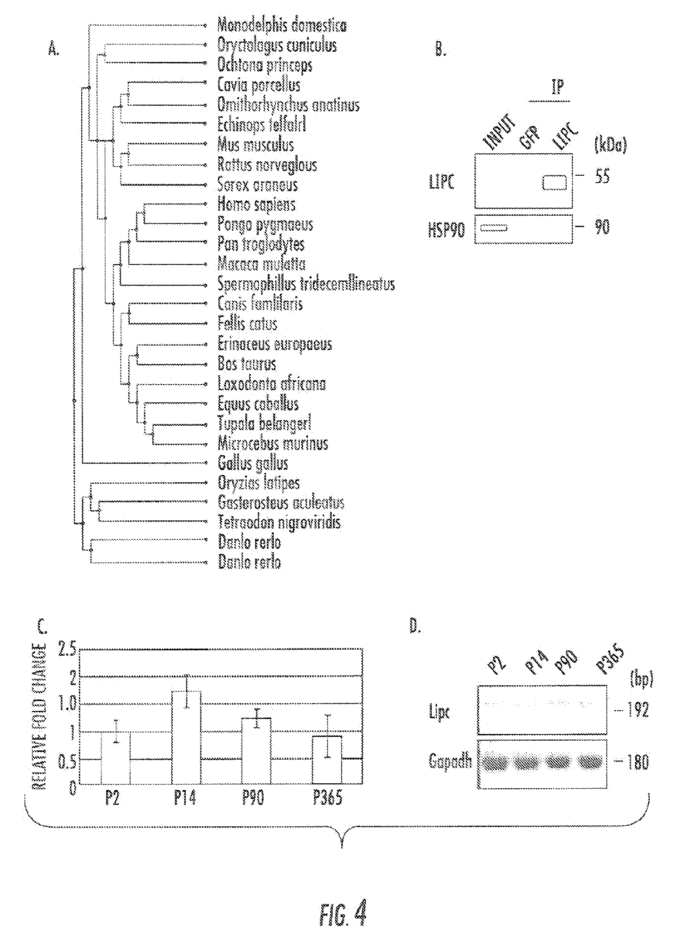

Sequence conservation for the LIPC gene in evolution, with approximately 75% amino
acid identity across mammals **(**Fig. **4a** **-** Phylogenetic tree), was found phylogenetically. We decided to investigate whether the LIPC protein product was expressed in the eye and if there were any significant changes to this expression over time. Despite LIPC being considered a hepatic enzyme, we were able to detect low-levels of LIPC protein in bovine retinas by immunoprecipitation using a monoclonal LIPC antibody. The bovine retinas did not show any presence of green fluorescent protein (GFP), whose antibody was also incubated and immunoblotted, but did not produce bands signifying their detectable existence **(****Fig**. **4b****).** Consistent with this result, we were also able to show that the LIPC RNA is present in the retinas of C57BL/6 mice across a broad range of ages **(**Fig. **4c****).** In **Figure 4d****,** the retinas of post-natal (P) 2, P14, P90, and P365 mice were examined using quantitative RT-PCR. The relative fold change of the LIPC RNA does appear to fluctuate, with the most expression appearing to occur during puberty transition. However, the expression level does appear to remain steady throughout the first year of life which should approximate the age of onset for AMD in humans. Consistent with our expression data from mouse retina, we were also able to amplify LIPC message from total RNA isolated both from human retinal epithelial transformed cells (RPE19) as well as from human eyes. Next, using a rabbit polyclonal antibody raised against amino acids 91-160 of human LIPC, we were able to demonstrate strong labeling of human macula and RPE/choroid and weaker labeling of peripheral retina at the appropriate molecular weight of ∼55 kDa observed in human liver **(**Fig. **5****).** Bands of mobility ∼80kD likely represent glycosylated hepatic lipase. This same antibody was then used to immunolocalize LIPC in sections of perfusion-fixed monkey retina **(**Fig. **6****).** Prominent labeling of all retinal neurons was observed, especially retinal ganglion cells of the central retina. However, Mueller glial cells were not labeled. RPE appeared to exhibit significant fluorescence signal beyond its intrinsic autofluorescence. Thus, our immunoblot and immunohistochemical data corroborate the findings of Tserentsoodol, et al., 2006 regarding the existence of intra-retinal proteins (e.g., ABCA1transporter, apoA1, SR-BI, SR-BII) that mediate lipid transport and processing.

We found a strong association between advanced AMD and the LIPC locus. LIPC (encoding hepatic triglyceride lipase) catalyzes hydrolysis of phospholipids, mono-, di-, triglycerides, acyl-CoA thioesters and is a critical enzyme in HDL metabolism.

Hepatic lipase also binds heparin and has the dual functions of triglyceride hydrolase and ligand/bridging factor for receptor-mediated lipoprotein uptake. LIPC has been shown to be expressed in the retina through our experiments, and CETP and ABCA1 have previously been shown to be expressed in the retina.

The pattern of results from the previously reported HDL loci is inconsistent with a straightforward correlation between HDL levels and AMD. If the association between LIPC and AMD is driven by this phenotypic correlation, then all SNPs associated with HDL would likely show some level of association with AMD. Additionally, the direction of the effects would be consistent. The genome-wide associated LIPC SNP has been shown to increase HDL levels, and our data suggests it decreases risk for AMD. In contrast, data from our scan suggest that the HDL raising allele of ABCA1 and CETP may increase the risk of AMD, although these results are not currently genome-wide significant. Overall, these observations suggest that the association with LIPC and the possible association with the other HDL loci may not be the direct result of serum HDL levels.

If the relationship of the LIPC polymorphism to AMD is not mediated by variation in HDL levels, alternative mechanisms may play a role. Specifically, hepatic lipase has been shown to have a major and complex impact on atherogenesis. Clinical and epidemiological studies have found that atherosclerotic cardiovascular risk factors are also risk factors for AMD. It has been proposed that the vascular intimae in atherosclerosis and Bruch's membrane in macular degeneration undergo similar agerelated changes. Such diseases may thus represent parallel responses to the tissue injury induced by multiple factors including genetic variation, improperly directed immune responses and oxidative stress. Whether LIPC genetic variation has parallel or distinct roles in AMD and atherogenesis remains to be determined.

The data in the epidemiologic literature regarding the association between serum HDL levels and AMD are conflicting. Some studies failed to find a relationship with HDL levels and macular degeneration. An increased risk of AMD, or a subphenotype of AMD, was associated with increased HDL levels. Three studies have shown an inverse relationship between risk of AMD and HDL levels, either decreased HDL levels in AMD cases in case-control studies, or decreased incidence of advanced AMD with higher HDL levels in prospective cohort studies. The T allele of rs10468017 in LIPC is protective for AMD and associated with higher HDL levels.

Another possibility regarding the role of the LIPC polymorphism in AMD involves the role of HDL as the major lipoprotein transporter of lutein and zeaxanthin. Reduced dietary intake of these two carotenoids has been associated with an increased risk of AMD. Variation in the uptake and transport into the retina of carotenoids by HDL has been implicated in AMD pathogenesis. Changes in HDL-related efficiency of carotenoid delivery is another possible mechanism by which LIPC variation could impact the risk of AMD. It is also interesting to note that drusen, deposits in the macula which are the hallmark of AMD, also contain cholesterol deposits. This LIPC locus greatly enhances our biological understanding, by opening up an entirely new pathway for consideration in the pathogenesis of AMD.

### METHODS

**Study Sample Descriptions.** The methods employed in this study conformed to the tenets of the Declaration of Helsinki, received approval from Institutional Review Boards, and informed consent was signed by all participants. Some methods have been described in detail previously. Cases had geographic atrophy or neovascular disease based on fundus photography and ocular examination (Clinical Age-Related Maculopathy Grading System (CARMS) stages 4 and 5).

Controls were unrelated to cases, 60 years of age or older, and were defined as individuals without macular degeneration, categorized as CARMS stage 1, based on fundus photography and ocular examination. Subjects were derived from ongoing AMD study protocols as described previously. The Tufts/MGH replication dataset was comprised of DNA samples from unrelated Caucasian individuals not included in the GWAS, including 868 advanced AMD cases and 410 examined controls who were identified from the same Tufts cohorts, and 379 unexamined MGH controls.

### Genotyping, Analysis, and Replication.

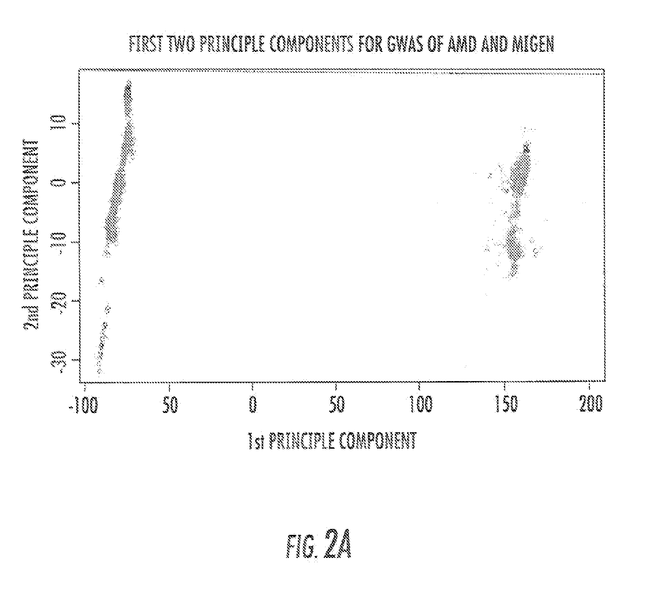

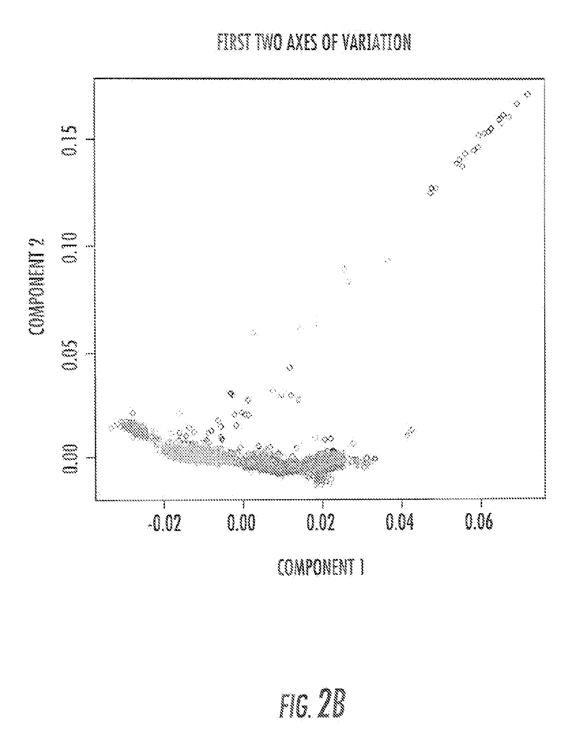

The GWAS genotyping and the Tufts/MGH follow-up replication genotyping were performed at the Broad and National Center for Research Resources (NCRR) Center for Genotyping and Analysis using the Affymetrix SNP 6.0 GeneChip and the Sequenom MassARRAY system for iPLEX assays, respectively. We started with a primary dataset of 1,057 cases and 558 examined controls and studied 906,000 genotyped SNPs and 946,000 CNVs using the Affymetrix 6.0 GeneChip which passed quality control filters. Then 43,562 SNPs were removed for low call rate, 4,708 were removed for failing Hardy-Weinberg test at 10-3, and 8,332 SNPs were removed because of failing a differential missing test between cases and controls at 10-3. Finally, 126,050 SNPs were removed for having allele frequency less than 1%, similar to other studies using this methodology. Thus, we evaluated 726,970 SNPs in this study in the discovery phase. We also removed 73 individuals for lower than expected call rate, resulting in 1,006 cases and 536 controls. All quality control steps were performed using PLINK. We conducted a preliminary χ2 association analysis to determine the extent to which population stratification and other biases were affecting the samples and observed a lambda of ∼1.05, indicating that the samples were generally well matched for population ancestry, with some minor inflation remaining (explanation and visual representation see **Figure 2A** **&** **2B****).** We added the MIGEN shared controls which were genotyped on the same Affymetrix 6.0 GeneChip product and conducted population stratification analyses using multi-dimensional scaling in PLINK. These analyses identified 27 cases, 12 AMD controls and 223 MIGEN controls for a total of 262 individuals which were outliers in the principal component analysis. The final genomic control lambda for the logistic regression included seven significant (for prediction of phenotype status) principal components as covariates and was 1.036 for 632,932 SNPs. This dataset was used for our official GWAS analysis.

We evaluated SNPs with *P*<10-3 from our GWAS discovery sample (n= 720 SNPs excluding previously associated regions) in the MPM GWAS. The exchange of top hits enabled us to use the two scans as primary replication efforts which enhanced the power of each study. We performed genotyping of all SNPs with combined *P*<10-4 using Sequenom iPLEX at the Broad NCRR Genotyping Center using our Tufts/MGH replication sample. Focusing on sites which continued to show association with *P*<10-4 after this local replication, we performed a third stage of replication in which our collaborators at JHU and Columbia University genotyped SNPs with ABI 7900 Taqman genotyping, samples from Washington University were genotyped using the Sequenom platform, and samples from University of Utah and Creteil, France were sent to JMS in Boston for genotyping at the Broad Institute, Cambridge, MA **(Table 1).**

### Copy number variation.

Using the Birdsuite family of algorithms, we called rare copy number variation and common copy number variation in 459 controls and 838 cases, after removing those individuals failing SNP clustering, that had an excessive number of singleton CNVs, or an excessive length of total length of singleton CNVs.

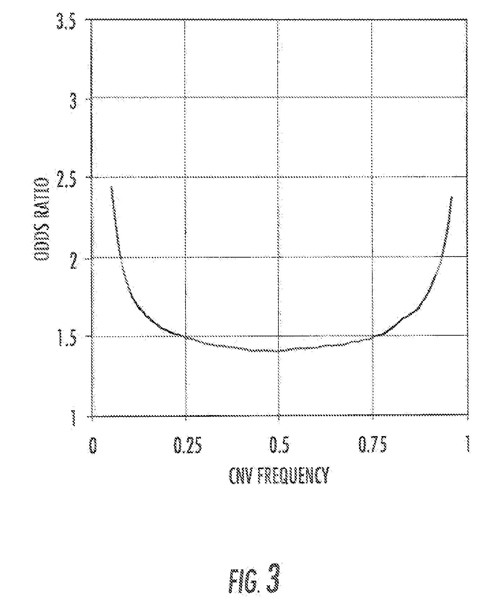

The study was well powered to detect CNV events **(****Figure 3****).** For rare variants, we identified all singleton copy number variants that were larger than 100 kb, had LOD scores greater than 10, and relied on at least 10 probes. We also looked at a smaller set if rare variants (>20 kb) that appeared in <1% of individuals, and used PLINK's permutation function to assess if there were any regions that had a significantly different numbers events overlapping cases versus controls. For common variants we examined 554 variants that had at least one allele <99%. We used logistic regression in PLINK to test if copy number significantly predicted case-control status.

### Multidimensional Scaling for Additional Controls.

To augment the control set, a subset of controls (n = 1409) from the Myocardial Infarction Genetics (MIGEN) Project was used. Briefly, MIGEN controls are ascertained across Europe, for absence of an MI event. These controls are unscreened for AMD, and so the utility of including them was assessed by examining the previously reported associations in the literature. Specifically, an assessment as to whether the loci at CFH, ARMS2, CFI, C3, CF/B2 showed more significant association to AMD upon expansion of the control sample was performed. The inclusion of these shared controls yielded a dramatic increase in the lambda (2.2). Multi-dimensional scaling was applied based on all pair-wise identity-by-state comparisons for all individuals. The first multi-dimensional scaling component separated out completely the shared controls from the initial dataset (Figure 2A). American populations can be matched to European populations (as long the European populations are diverse), so this complete delineation between the shared controls and the original dataset was due to technical bias between the two datasets. Moving the call rate threshold from 95% to 99% dramatically reduced the lambda (1.22), but still, apparent population stratification effects persisted. Multi-dimensional scaling was again applied to the IBS matrix, examining the first 10 axes of variation. The first axis of variation no longer classified the cases and controls. The second axis of variation identified a handful of individuals who were apparently either demonstrating high levels of technical bias or were from a different ancestral background (Figure 2B). Finally, the axes of variation were examined to determine whether they significantly predicted case or control status across the genome at an average P-value less than 0.05. Doing so yielded 7 axes of variation and a lambda of 1.036, comparable to the initial study lambda, with an expanded sample size.

**Table 1. Age-related macular degeneration grade, gender and age information for samples.**

| | **Tufts/MGH Affy** | **UM ILMN** | **Tufts/MGH Replication** | **JHU** | **NY** |
|---|---|---|---|---|---|
| | N (%) | N (%) | N (%) | N (%) | N (%) |
| **AMD Grade** | | | | | |
| 1 | 524 (35) | 1138 (44) | 410 (32) | 136 (22) | 368 (33) |
| 4 | 269 (18) | 415 (16) | 246 (19) | 95 (15) | 211 (19) |
| 5 | 710 (47) | 1037 (40) | 622 (49) | 389 (63) | 524 (48) |
| | | | | | |

| **Gender (N %)** | | | | | |
|---|---|---|---|---|---|
| M | 691 (46) | 1062 (41) | 513 (40) | 209 (34) | 418(40) |
| F | 812 (54) | 1528 (59) | 765 (60) | 411 (66) | 632 (60) |
| | | | | | |

| **Mean Age by AMD grade** | | | | | |
|---|---|---|---|---|---|
| 1 | 76 | 74 | 73 | 74 | 75 |
| 4 | 81 | 78 | 79 | 76 | 80 |
| 5 | 80 | 80 | 80 | 77 | 79 |

Tufts/MGH Affy represents the genome-wide association scan using the Affymetrix 6.0 platform from Tufts Medical Center, Tufts University School of Medicine, without the MIGEN controls included; Tufts/MGH Replication represents the follow up replication pool at MGH/Tufts; UM ILMN represents the genome-wide association scan using the Illumina 322 platform from the University of Michigan; JHU represents the Johns Hopkins University sample replication, and NY represents the Columbia University sample replication. AMD Grading System: grade 1 represents individuals with no drusen or a few small drusen, 4 represents individuals with central or non-central geographic atrophy ("advanced dry type"), and 5 represents individuals with neovascular disease ("advanced wet type").

**Table 2. The evolution of sample size as a function of the quality control process.**

| | Cases | Controls | Change to Sample Size | #SNPs |
|---|---|---|---|---|
| Initial Sample | 1057 | 558 | - | 909622 |
| Initial Dataset Cleaning | 1006 | 536 | -73 | 726970 |
| Addition of Shared Controls | 1006 | 1944 | 1409 | 707919 |
| Removal of Clustering Outliers | 979 | 1709 | -262 | 632932 |

Each step represents a cleaning stage. The initial sample represents all samples genotyped. The initial dataset cleaning encompasses HWE, call rate, differential missingness between cases and controls, and minor allele frequency threshold. Adding in shared controls, the call rate and MAF thresholds were reapplied. For the final stage, call rate of 99% was required as was the removal of individuals who did not cluster with the majority of the sample.

**Table 3. Genome-wide association study results for previously reported AMD loci.**

| Gene | Associated SNP | Best Proxy SNP | Distance | r² | Chr | Position | Tufts | Tufts/MGH *P*-value | MPM *P*-value | Meta *P* |
|---|---|---|---|---|---|---|---|---|---|---|
| CFH | rs1061170 | rs572515 | 12976 | 0.654 | 1 | 194912884 | 2.19E-43 | 3.84E-56 | 5 76E-63 | 4.00E-117 |
| CFH | rs1410996 | rs10737680 | 17478 | 1 | 1 | 194946078 | 6.63E-45 | 3.52E-48 | 6.66E-66 | 1.66E-111 |
| CFI | rs10033900 | rs7690921 | 80321 | 0.403 | 4 | 110798195 | 3.34E-04 | 1.26E-05 | 1.69E-04 | 9.05E-09 |
| CFB/C2* | rs641153 | rs522162 | 5737 | 1 | 6 | 32027896 | 7.86E-07 | 1.07E-07 | 5.96E-15 | 1 73E-20 |
| Variant near C3; | rs2230199 | rs2241392 | 32654 | 0.08 | 19 | 6636983 | 9.21E-03 | 8.48E-04 | 4.15E-06 | 1.96E-08 |
| ARMS2/HTRA1 | rs10490924 | rs10490924 | 0 | 1 | 10 | 124204438 | 2.06E-41 | 1.32E-60 | 2.36E-60 | 4.53E-119 |

| | | | | | | | | | | |
|---|---|---|---|---|---|---|---|---|---|---|
| *Second independent variant at C2 was not proxied by Affymetrix 6.0 | | | | | | | | | | |

Results in the GWA studies for SNPs which have previously been replicated as associated with AMD. SNP, single nucleotide polymorphism; r2, correlation coefficient between best proxy SNP on Affymetrix 6.0 platform and the previously associated SNP; Chr, chromosome; Position, base pair position in NCBI 35 or 36, Tufts represents the discovery genome-wide scan, without the addition of the MIGEN shared controls, Tufts/MGH includes the Tufts Medical Center, Tufts University School of Medicine sample plus the MIGEN shared controls, MPM refers to Michigan/Penn/Mayo shared results from the genome-wide association scan, and the Meta analysis refers to the combined P-value for the meta-analysis of Tufts/MGH and MPM genome-wide scans.

**Table 4. Results of previously published putative SNP associations with AMD in our genome wide association study.**

| Gene | SNP | Reported P-vale | Paper | Chr | Position | Best Proxy | R² | Position | Ref Allele | OR | P-value |
|---|---|---|---|---|---|---|---|---|---|---|---|
| TLR3 | rs3775291 | 543E-04 | Yang Z et al. NEJM 2008⁴ | 4 | 187241068 | rs3775291 | 1 | 187241068 | T | 0.99 | 0.87 |
| | Grade 4 only | | | | | | | | 0.96 | 0.71 | |
| ELOVL4 | rs3812153 | 1.00E-04 | Conley YP et al. HMG 2005⁵ | 6 | 80683094 | rs2991 | 0.94 | 80681375 | C | 0.89 | 0.22 |
| PON1 | rs662 | 9.00E-03 | Ikeda T et al. AJO 2001⁶ | 7 | 94775382 | rs2057681 | 1 | 94776193 | G | 0.98 | 0.75 |
| SERPING1 | rs2511989 | 6.00E-07 | Ennis S et al. Lancet 2008⁷ | 11 | 57134901 | rs2511989 | 1 | 57134901 | T | 1.02 | 0.78 |
| LRP6 | rs7294695 | 4.00E-03 | Haines JL et al. IOVS 2006 | 12 | 12214885 | rs7294695 | 1 | 12214885 | G | 1.02 | 0.76 |
| APOE | rs429358, rs7412 | 4.00E-03 | Bojanowski CM et al Env and Mol Mutagenesis 2006 | 19 | 501103781, 50103919 | rs4420638 | N/A | 50114786 | C | 0.84 | 0.13 |
| PEDF | rs1136287 | 5.00E-04 | Lin J-M et al. AJO 2008⁹ | 17 | 1620026 | rs1136287 | 1 | 1620026 | C | 1.05 | 0.40 |
| CX3CR1 | rs3732378 | 0.04 | Tuo J et al FASEB J 2004 | 3 | 39282166 | rs3732378 | 1 | 39282166 | A | 0.91 | 0.09 |
| VEGF | rs833070 | 0.001 | Haines JL et al IOVS 2006 | 6 | 43850604 | rs833070 | 1 | 43850604 | T | 0.95 | 0.11 |
| SOD2 | rs4880 | 0.0005 | Kimura K et al Am J Ophthal 2000 | 6 | 160033862 | rs4880 | 1 | 160033862 | A | 1.11 | 0.19 |
| TLR4 | rs4986790 | 0.025 | Zareparsl S et al Hum Mol Genet 2005 | 9 | 119515123 | rs4986790 | 1 | 119515123 | A | 0.92 | 0.52 |

| | | | | | | | | | | | |
|---|---|---|---|---|---|---|---|---|---|---|---|
| Results in our GWAS for loci reported to be associated with AMD in one or more articles with a p value < 0.001 are shown. We fail to replicate the *TLR3, ELOVL4, PON1, SERPING1, LRP6, PEDF4, and APOE* results. Grade 4 represents geographic atrophy, the focus of the original *TLR3* association report.(4) R2=correlation coefficient; Ref=referent; OR=odds ratio; N/A= not available, rs429358 and rs7412 are not in HapMap so r2 could not be calculated. †The data analyzed for APOE was from the initial case-control dataset (before MIGEN controls were added) as rs4420638 did not pass our quality control threshold for genotype missingness to be included in the shared control dataset. | | | | | | | | | | | |

**Table 5. Results of replication and meta-analysis.**

| -log10(P-val) | Tufts/MGH | Tufts/MGH+UM | Tufts/MGH+UM+Tufts Replication |
|---|---|---|---|
| <3 | 378 | 81 | 8 |
| <4 | 41 | 30 | 15 |
| <5 | 2 | 20 | 4 |
| <6 | 0 | 0 | 1 |

This table represents the number of loci with a P-value less than the threshold excluding known loci. As we increase the total sample size, the significance for our likely SNP continues to move in the same direction.

**Table 6. Replication panel test for heterogeneity in the odds ratio for the disease/SNP association to rs10468017.**

| **SNP** | **CHISQ** | **DF** | **P** | **Panel** |
|---|---|---|---|---|
| rs10468017 | 13.41 | 5 | 0.01982 | ALL |
| rs10468017 | 2.197 | 1 | 0.1383 | SQ-AFFY |
| rs10468017 | 0.1137 | 1 | 0.736 | COL |
| rs10468017 | 4.237 | 1 | 0.03955 | FR |
| rs10468017 | 0.08777 | 1 | 0.767 | JHU |
| rs10468017 | 9.002 | 1 | 0.002697 | UT |
| rs10468017 | 0.000173 | 1 | 0.9895 | WASH |

The SNP tested was rs10468017. CHISQ = The chi-square for each panel versus the other panels as implemented in PLINK. DF = Degree of Freedom for each test. P = p-value where the lower numbers represents greater heterogeneity. Panel = The samples tested in each phase of our meta-analysis.

**Table 7a. Meta-analysis association results from two genome-wide association scans and replication genotyping.**

| SNP | Chr | BP | A1:A2 | Tufts/MGH | UM P | Tufts/MGH Rep P | Combined P | JHU P | Combined P | COL P | Combined P |
|---|---|---|---|---|---|---|---|---|---|---|---|
| rs493258 | 15 | 56475172 | T:C | 4.53E-05 | 9.98E-03 | 9.01E-02 | 8.47E-07 | 1.51 E-02 | 4.07E-08 | 1.47E-02 | 2.18E-09 |
| rs6531212 | 2 | 20201501 | T:C | 2.41E-04 | 3.90E-02 | 1.54E-02 | 2.32E-06 | 9.13E-01 | 2.07E-05 | 4.52E-01 | 1.58E-04 |
| rs7626245 | 3 | 101053451 | C:G | 9.61E-05 | 3.32E-02 | 5.78E-02 | 3.46E-06 | 2.18E-01 | 1.99E-04 | 5.84E-01 | 2.20E-04 |
| rs11755724 | 6 | 7063989 | A:G | 5.74E-04 | 4.84E-02 | 1.00E-02 | 4.14E-06 | 8.27E-01 | 1.83E-05 | | |
| rs17628762 | 18 | 34125717 | A:C | 6.32E-04 | 2.05E-03 | 3.82E-01 | 9.84E-06 | 6.09E-01 | 1.36E-04 | | |
| rs195484 | 6 | 116341661 | C:T | 1.46E-04 | 5.75E-02 | 7.23E-02 | 1.14E-05 | | | | |
| rs4628134 | 6 | 116466834 | C:T | 4.30E-04 | 1.08E-02 | 2.01E-01 | 1.32E-05 | 3.15E-01 | 1.17E-05 | 3.69E-01 | 9.12E-06 |
| rs1931897 | 6 | 116546700 | C:A | 3.09E-04 | 4.17E-03 | 4.35E-01 | 1.32E-05 | | | | |
| rs12210252 | 6 | 116584186 | C:A | 1.54E-04 | 2.86E-03 | 7.66E-01 | 1.73E-05 | | | | |
| rs17629141 | 18 | 34142262 | A:G | 6.43E-04 | 2.56E-03 | 4.89E-01 | 1.82E-05 | | | | |
| rs2036936 | 18 | 34126301 | G:C | 8.29E-04 | 2.05E-03 | 6.39E-01 | 3.00E-05 | | | | |
| rs564253 | 6 | 116531407 | T:C | 6.59E-04 | 2.07E-03 | 6.99E-01 | 3.06E-05 | | | | |
| rs12196141 | 6 | 116596243 | G:A | 3.30E-04 | 3.05E-03 | 8.09E-01 | 3.51E-05 | | | | |
| rs10739343 | 9 | 114121319 | A:G | 8.37E-04 | 2.58E-02 | 1.47E-01 | 3.52E-05 | | | | |
| rs1468678 | 9 | 128236471 | A:G | 7.19E-05 | 2.22E-01 | 5.73E-02 | 3.64E-05 | | | | |
| rs2803544 | 10 | 88396729 | A:T | 2.77E-04 | 1.81E-02 | 3.78E-01 | 3.85E-05 | | | | |
| rs3748391 | 16 | 85846518 | G:T | 7.30E-04 | 6.57E-02 | 7.01E-02 | 4.11E-05 | | | | |
| rs2803590 | 10 | 88362973 | A:G | 3.20E-04 | 2.33E-02 | 3.24E-01 | 4.36E-05 | 2.49E-01 | 1.16E-03 | | |
| rs12637095 | 3 | 119674993 | T:A | 9.39E-05 | 3.41 E-02 | 4.97E-01 | 5.54E-05 | 7.16E-01 | 4.28E-04 | | |
| rs509859 | 6 | 116529937 | T:G | 5.18E-04 | 2.13E-03 | 8.75E-01 | 8.42E-05 | 1.92E-02 | 5.42E-06 | 2.82E-01 | 3.33E-06 |
| rs3019878 | 8 | 123539159 | A:G | 2.59E-05 | 8.05E-02 | 6.15E-01 | 9.59E-05 | | | | |
| rs887656 | 9 | 128237337 | T:C | 7.83E-05 | 2.22E-01 | 1.61E-01 | 1.10E-04 | | | | |
| rs401758 | 5 | 80318883 | T:C | 3.20E-04 | 5.12E-02 | 3.53E-01 | 1.12E-04 | | | | |
| rs2059883 | 16 | 85856833 | C:T | 9.35E-04 | 6.42E-02 | 1.72E-01 | 1.21E-04 | | | | |
| rs1511454 | 4 | 23191579 | G:A | 7.35E-04 | 1.67E-02 | 5.81 E-01 | 1.38E-04 | 4.55E-01 | 1.56E-03 | | |
| rs1529777 | 15 | 92694176 | G:A | 1.79E-04 | 2.13E-01 | 1.58E-01 | 1.67E-04 | | | | |
| rs10089310 | 8 | 128550166 | A:T | 4.91E-04 | 2.13E-03 | 4.89E-01 | 2.38E-04 | 1.18E-01 | 6.88E-03 | | 6.88E-03 |
| rs991386 | 5 | 61098382 | C:T | 9.40E-05 | 5.96E-03 | 3.82E-01 | 2.69E-04 | | | | |
| rs12248838 | 10 | 63565617 | G:C | 2.80E-04 | 2.30E-01 | 1.90E-01 | 2.99E-04 | | | | |
| rs3924739 | 5 | 61117491 | C:T | 1.63E-04 | 4.32E-03 | 3.24E-01 | 3.57E-04 | | | | |
| rs1457239 | 2 | 160722499 | G:C | 4.26E-05 | 2.14E-01 | 6.36E-01 | 4.56E-04 | | | | |
| rs6449556 | 5 | 61117558 | A:G | 2.60E-04 | 6.87E-03 | 3.71E-01 | 5.61 E-04 | | | | |
| rs6862305 | 5 | 61091141 | C:T | 1.48E-04 | 9.30E-03 | 3.37E-01 | 5.78E-04 | | | | |
| rs4883193 | 12 | 8914959 | A:C | 5.04E-04 | 4.39E-02 | 9.04E-01 | 6.09E-04 | | | | |
| rs487766 | 15 | 56481152 | C:T | 1.43E-04 | 6.33E-01 | 1.47E-01 | 8.19E-04 | | | | |
| rs16993349 | 20 | 7214858 | G:A | 7.36E-05 | 3.37E-01 | 6.14E-01 | 1.08E-03 | | | | |
| rs7704053 | 5 | 7832503 | A:G | 2.55E-04 | 7.02E-02 | 8.28E-01 | 1.18E-03 | | | | |
| rs4688116 | 3 | 119678291 | G:T | 2.77E-04 | 5.15E-02 | 6.66E-01 | 1.34E-03 | | | | |
| rs2417019 | 9 | 128261827 | C:T | 1.29E-04 | 1.77E-01 | 9.46E-01 | 1.34E-03 | | | | |
| rs408686 | 2 | 39030824 | C:A | 9.02E-06 | 3.48E-01 | 7.81 E-01 | 1.59E-03 | | | | |
| rs4447616 | 2 | 166871909 | C:T | 5.84E-04 | 1.12E-01 | 9.94E-01 | 2.02E-03 | | | | |
| rs16908453 | 8 | 139259831 | A:G | 4.47E-04 | 1.39E-01 | 8.47E-01 | 3.04E-03 | | | | |
| rs11954345 | 5 | 167242558 | G:A | 3.74E-04 | 1.38E-01 | 7.67E-01 | 3.25E-03 | | | | |
| rs6126970 | 20 | 51848514 | T:C | 3.25E-04 | 5.67E-02 | 3.89E-01 | 3.26E-03 | | | | |
| rs2205193 | 14 | 76407098 | G:A | 7.92E-05 | 5.12E-01 | 8.19E-01 | 3.34E-03 | | | | |
| rs7957619 | 12 | 108498262 | A:G | 1.97E-04 | 6.16E-01 | 6.19E-01 | 4.57E-03 | | | | |
| rs869141 | 2 | 216242628 | G:C | 2.53E-04 | 8.89E-01 | 4.02E-01 | 6.05E-03 | | | | |
| rs7258519 | 19 | 19917348 | C:T | 4.31E-04 | 2.65E-01 | 7.86E-01 | 6.82E-03 | | | | |
| rs1808882 | 3 | 144553442 | A:G | 1.15E-03 | 5.63E-01 | 4.87E-01 | 7.05E-03 | | | | |
| rs6022766 | 20 | 51861627 | A:C | 5.92E-04 | 6.29E-02 | 2.44E-01 | 7.69E-03 | | | | |
| rs11767862 | 7 | 8840342 | T:C | 9.66E-05 | 8.28E-01 | 4.75E-01 | 9.01E-03 | | | | |
| rs1056104 | 2 | 38862453 | A:G | 7.05E-04 | 3.92E-01 | 8.39E-01 | 1.24E-02 | | | | |
| rs11996500 | 8 | 16618572 | G:A | 3.76E-04 | 3.04E-01 | 5.16E-01 | 1.29E-02 | | | | |
| rs4129669 | 1 | 56062111 | A:G | 3.97E-04 | 6.70E-01 | 3.40E-01 | 1.71E-02 | | | | |
| rs6960445 | 7 | 21305064 | A:G | 1.33E-04 | 4.59E-01 | 3.40E-01 | 1.82E-02 | | | | |
| rs12411257 | 1 | 56055920 | T:C | 1.47E-04 | 5.89E-01 | 5.67E-01 | 2.26E-02 | | | | |
| rs2290445 | 2 | 39068325 | A:C | 1.48E-04 | 5.82E-01 | 3.75E-01 | 2.64E-02 | | | | |
| rs11068670 | 12 | 116666552 | G:C | 1.72E-04 | 9.62E-02 | 2.02E-02 | 3.04E-02 | | | | |
| rs33445 | 5 | 52317671 | G:A | 2.28E-04 | 5.30E-01 | 5.76E-01 | 3.15E-02 | | | | |
| rs11621648 | 14 | 51734555 | T:A | 6.02E-05 | 9.20E-01 | 5.56E-01 | 3.55E-02 | | | | |
| rs33446 | 5 | 52317788 | C:G | 1.98E-04 | 5.30E-01 | 6.88E-01 | 3.61 E-02 | | | | |
| rs10803311 | 1 | 14939531 | C:T | 2.08E-04 | 2.21E-01 | 2.09E-02 | 6.11E-02 | | | | |
| rs10141408 | 14 | 93847114 | T:C | 2.50E-04 | 7.28E-01 | 5.03E-01 | 8.98E-02 | | | | |
| rs10935124 | 3 | 135884796 | A:C | 4.61E-04 | 7.83E-01 | 1.46E-01 | 1.12E-01 | | | | |
| rs9901699 | 17 | 76042005 | G:A | 1.55E-04 | 4.44E-01 | 5.92E-01 | 1.14E-01 | | | | |
| rs2197714 | 3 | 135905364 | A:T | 3.63E-04 | 9.44E-01 | 1.74E-01 | 1.43E-01 | | | | |
| rs9508553 | 13 | 29255155 | C:G | 9.80E-04 | 3.63E-01 | 1.45E-02 | 1.74E-01 | | | | |
| rs1562483 | 3 | 135915620 | G:T | 7.40E-04 | 9.21E-01 | 1.84E-01 | 1.79E-01 | | | | |
| rs10520589 | 15 | 83811349 | C:A | 7.71E-04 | 3.77E-01 | 5.34E-01 | 2.02E-01 | | | | |
| rs168331 | 5 | 141355147 | C:A | 2.92E-04 | 3.83E-01 | 3.66E-01 | 2.13E-01 | | | | |
| rs17143361 | 16 | 7295259 | T:C | 7.00E-04 | 9.45E-02 | 7.97E-01 | 3.40E-01 | | | | |
| rs17449231 | 7 | 16966165 | A:G | 4.40E-04 | 2.71E-01 | 1.10E-03 | 6.93E-01 | | | | |
| rs3915556 | 13 | 99522530 | T:C | 2.97E-05 | 3.12E-01 | 1.44E-03 | 7.26E-01 | | | | |
| rs12549290 | 8 | 15999123 | A:C | 6.92E-04 | 1.03E-02 | 7.42E-01 | 7.48E-01 | | | | |

A weighted Z score approach for the combination of the association information. These weights were calibrated on the theoretical power for a balanced case-control study design. Combined P value refers to the combination of the Tufts/MGH, UM, and Replication datasets. Final combined P-value refers to a total meta-analysis across the aforementioned sites and the samples from JHU. SNP, single nucleotide polymorphism; Chr, chromosome; Position, base pair position in NCBI 36.1 = chromosome, A1, Reference Allele for Analysis on + strand; A2, Second Allele of SNP.

**Table 7b. Association results for previously reported AMD genes.**

| Gene | Associated SNP | Best Proxy SNP | Distance | r² | Chr | Position | Tufts | Tufts/MGH P-value | | UMP-value | | Meta P | |
|---|---|---|---|---|---|---|---|---|---|---|---|---|---|
| CFH | rs1061170 | rs572515 | 12976 | 0.654 | 1 | 194912884 | 2.19E-43 | 3.84E-56 | | 5.76E-63 | | 4.00E-117 | |
| CFH | rs1410996 | rs10737680 | 17478 | 1 | 1 | 194946078 | 6.63E-45 | 3.52E-48 | | 6.66E-66 | | 1.66E-111 | |
| CFI | rs10033900 | rs7690921 | 80321 | 0.403 | 4 | 110798195 | 3.34E-04 | 1.26E-05 | | 1.69E-04 | | 9.05E-09 | |
| CFB/C2* | rs641153 | rs522162 | 5737 | 1 | 6 | 32027896 | 7.86E-07 | 1.07E-07 | | 5.96E-15 | | 1.73E-20 | |
| NEAR C3; NOT C3 Variant | rs2230199 | rs2241392 | 32654 | 0.08 | 19 | 6636983 | 9.21 E-03 | 8.48E-04 | | 4.15E-06 | | 1.96E-08 | |
| ARMS2/HTRA1 | rs10490924 | rs10490924 | 0 | 1 | 10 | 124204438 | 2.06E-41 | 1.32E-60 | | 2.36E-60 | | 4.53E-119 | |
| * Second independent variant at C2 was not proxied by Affymetrix 6.0 | | | | | | | | | | | | | |

The association results for SNPs which have previously been consistently replicated as associated with AMD. SNP, single nucleotide polymorphism; r², correlation coefficient between best proxy SNP on Affymetrix 6.0 platform and the previously associated SNP; Chr, chromosome; Position, base pair position in NCBI 35 or 36, Tufts represents the discovery genome-wide scan, without the addition of the MIGEN shared controls, Tufts/MGH includes the Tufts Medical Center, Tufts University School of Medicine sample plus the MIGEN shared controls, UM refers to the University of Michigan's shared results from the genome-wide association scan, and the Meta-analysis refers to the combined P-value for the meta-analysis of Tufts/MGH and UM genome-wide scans.

**Table 8. Meta-analysis association results from genome-wide association study discovery sample and replication genotyping.**

| SNP | Chr | Position | A1:A2 | MAF | # Associated SNPs | Tufts/MGH *P* | Tufts/MGH Repl *P* | MPM *P* | Repl Cohort *P* | Meta Z score | Meta OR | 95%Cl | Final *P* | |
|---|---|---|---|---|---|---|---|---|---|---|---|---|---|---|
| rs9621532 | 22 | 31414511 | C:A | 3.5% | 1 | 4.47E-02 | 328E-02 | N/A | 6.53E-08 | 5.897 | 0.618 | 0.526 - 0.725 | 3.71E-09 | ^{abcde} |
| rs493258 | 15 | 56475172 | T:C | 44.8% | 3 | 4.53E-05 | 1.44E-01 | 9.98E-03 | 2.36E-03 | 5.650 | 0.862 | 0.818 - 0.907 | 1.61E-08 | ^{abcde} |
| rs11755724 | 6 | 7063989 | A:G | 35.5% | 1 | 5.74E-44 | 1.34E-02 | 4.84E-02 | 3.12E-02 | 4.894 | 0.868 | 0.819 - 0.918 | 9.88E-07 | ^{abcde} |
| rs13095226 | 3 | 100878962 | C:T | 11.6% | 3 | 5.03E-04 | 2.38E-01 | 1.16E-01 | 3.35E-03 | 4.708 | 1.236 | 1.131 - 1.349 | 2.50E-06 | ^{abcde} |
| rs509859 | 6 | 116529937 | T:G | 38.2% | 5 | 5.18E-04 | 8.41E-01 | 2.13E-03 | 1.40E-01 | 4.124 | 0.924 | 0.890 - 0.959 | 3.73E-05 | ^{abcde} |
| rs3748391 | 16 | 85846518 | G:T | 471% | 2 | 7.30E-04 | 7.28E-02 | 6.57E-02 | 2.24E-01 | 3.946 | 1.117 | 1.057 - 1.180 | 7.95E-05 | ^{abcde} |
| rs12637095 | 3 | 119674993 | T:A | 19.6% | 2 | 9.39E-05 | 4.01E-01 | 3.41E-02 | 4.07E-01 | 3.716 | 0.895 | 0.844 - 0.949 | 2.03E-04 | ^{abcde} |
| rs10739343 | 9 | 114121319 | A:G | 20.3% | 1 | 8.37E-04 | 1.23E-01 | 2.58E-02 | 9.60E-01 | 3.412 | 0.898 | 0.844 - 0.955 | 6.45E-04 | ^{abc} |
| rs17628762 | 18 | 34125717 | A:C | 36.5% | 3 | 6.32E-04 | 4.14E-01 | 2.05E-03 | 9.94E-01 | 3.408 | 1.070 | 1.029 - 1.111 | 6.53E-04 | ^{abcde} |
| rs6531212 | 2 | 20201501 | T:C | 331% | 1 | 2.41 E-04 | 8.33E-03 | 3.90E-02 | 3.48E-03 | 3.163 | 0.926 | 0.882 - 0.971 | 1.56E-03 | ^{abcde} |
| rs10089310 | 8 | 128550166 | A:T | 10.6% | 1 | 4.91E-04 | 5.74E-01 | 2.13E-03 | 8.75E-01 | 2.807 | 1.102 | 1.029 - 1.178 | 5.01E-03 | ^{abcde} |
| rs2803544 | 10 | 88396729 | A:T | 14.2% | 3 | 2.77E-04 | 3.39E-01 | 1.81E-02 | 3.87E-01 | 2.683 | 1.076 | 1.019 - 1.134 | 7.29E-03 | ^{abcde} |

| | | | | | | | | | | | | | | |
|---|---|---|---|---|---|---|---|---|---|---|---|---|---|---|
| SNP: single nucleotide polymorphism; Chr: chromosome; Position: base pair position in NCBI 36.1; A1: Minor Allele; A2: Major Allele; MAF: Minor Allele Frequency; # Associated SNPs: number of other SNPs in the region showing association in our discovery scan; Tufts/MGH *P*: *P*-value of our original GWAS scan; Tufts/MGH Repl *P*: *P*-value of Tufts/MGH replication cohort; MPM *P*: *P*-value of Michigan/Penn/Mayo GWAS; Repl Cohort *P*: *P*-value of total combined weighted average Z score meta-analysis across the individual replication cohort sites from (a) JHU (Johns Hopkins University), (b) COL (Columbia University), (c) UT (University of Utah), (d) FR-CRET (Hopital Intercommunal de Creteil), and (e) WASH U (Washington University) as denoted; Meta Z score: weighted Z score used to combine the association from all cohorts calibrated on the theoretical power for a balanced case-control study design; Meta OR: odds ratio for AMD combining all cohorts, comparing the minor allele to the major allele; 95% CI: confidence interval for Meta OR; Final *P*: *P*-value of allcohorts listed in the Table plus the replication cohorts based upon the Meta Z score. | | | | | | | | | | | | | | |

**Table 9. Association results for the LIPC functional variant rs10468017.**

| | **rs10468017** | | | | | | | |
|---|---|---|---|---|---|---|---|---|
| | | | | | | | | |
| | Tufts/MGH-NoMIGEN | Tufts/MGH Replication | JHU | COL | UT | FR-CRET | WASH-U | Meta Analysis Final |
| **Study Weight (case:control = 1):** | 713 | 827 | 653 | 479 | 458 | 214 | 381 | 3725 |
| **Minor Allele (Frequency Affected / Frequency Unaffected):** | T (23.9% / 29.7%) | T (25.8% / 31.0%) | T (25.6% / 29.7%) | T (25.2% / 29.3%) | T (29.3% / 27.2%) | T (25.4% / 35.3%) | T (26.0% / 29.3%) | T (25.8% / 30.0%) |
| **Z-score:** | 3.582 | 3.233 | 2.321 | 1.924 | -1.008 | 3.251 | 1.573 | 5.681 |
| **Odds Ratio:** | 0.740 | 0.777 | 0.814 | 0.819 | 1.111 | 0.625 | 0.833 | 0.820 |
| **Lower 95% CI** | 0.628 | 0.667 | 0.683 | 0.668 | 0.905 | 0.470 | 0.663 | 0.766 |
| **Upper 95% CI** | 0.873 | 0.905 | 0.968 | 1.004 | 1.363 | 0.829 | 1.046 | 0.878 |
| **P-value:** | 3.42E-04 | 1.23E-03 | 2.03E-02 | 5.43E-02 | 0.3131 | 1.15E-03 | 0.1158 | 1.34E-08 |

Association results based upon six replication cohorts for a known functional variant of LIPC, rs10468017, also shown to be associated with HDL levels. Tufts/MGH-NoMIGEN: our discovery GWAS cohort without the MIGEN controls; Tufts/MGH Replication: local replication cohort; JHU: replication cohort at Johns Hopkins University; COL: replication cohort at Columbia; UT: replication cohort at University of Utah; FR-CRET: replication cohort at Hopital Intercommunal de Creteil; WASH-U: replication cohort at Washington University; Weight: the effective sample size of each cohort if the ratio between cases and controls was equal to one based upon actual samples listed in **S1.** Z-score: weighted average and direction of signal; Odds Ratio: weighted average odds ratio for the HDL-raising allele T compared to the major allele; 95% CI: confidence intervals for the odds ratio; P-value: P-value calculated from sum of weighted average Z score.

**Table 10. Association between AMD and previously reported HDL genetic loci.**

| Gene(s) of interest within or near associated interval | Chr | Position | SNP | HDL-raising allele | Minor Allele | MAF | Z-score | Meta *P* | *OR* (*Minor Allele)* | *95%Cl* | |
|---|---|---|---|---|---|---|---|---|---|---|---|
| ABCA1 | 9 | 106704122 | rs1683025 | C | T | 21.4% | 3.298 | 9.73E-04 | 0.774 | 0.664 - 0.901 | ^{abcdefg} |
| CETP | 16 | 55545545 | rs3764261 | A | A | 35.1% | 3.193 | 1.41E-03 | 1.119 | 1.044 - 1.198 | ^{abcdefg} |
| FADS1-3 | 11 | 61327359 | rs174547 | T | C | 32.5% | 2.213 | 2.69E-02 | 0.934 | 0.878 - 0.992 | ^{abcefg} |
| LPL | 8 | 19888502 | rs12678919 | G | G | 10.9% | 1.794 | 0.073 | 0.851 | 0.713 - 1.015 | ^{bed} |
| TTC39B | 9 | 15279578 | rs471364 | T | C | 10.5% | 1.405 | 0.60 | 1.156 | 0.944 - 1.415 | ^{abcef} |
| ANGPTL4 | 19 | 8375738 | rs2967605 | C | T | 20.5% | 1.104 | 0.270 | 1.109 | 0.922 - 1.332 | ^{abcef} |
| LIPG | 18 | 45421212 | rs4939883 | C | T | 16.2% | 1.052 | 0.293 | 0.958 | 0.883 - 1.038 | ^{abcef} |
| MMAB MVK | 12 | 108379551 | rs2338104 | G | C | 47.0% | 0.962 | 0.336 | 0.943 | 0.835 - 1.063 | ^{abeef} |
| LCAT | 16 | 66459571 | rs2271293 | A | A | 13.1% | 0.724 | 0.469 | 1.041 | 0.934 - 1.159 | ^{abcefg} |
| APOA1-APOC3-APOA4-APOA5 | 11 | 116154127 | rs964184 | C | G | 13.5% | 0.503 | 0.615 | 0.974 | 0.880 - 1.077 | ^{abcef} |
| PLTP | 20 | 44009909 | rs7679 | T | C | 18.1% | 0.395 | 0.693 | 1.016 | 0.940 - 1.097 | ^{abcefg} |
| HNF4A | 20 | 42475778 | rs1800961 | C | T | 3.1% | 0.078 | 0.938 | 0.989 | 0.744 - 1.312 | ^{abcef} |
| GALNT2 | 1 | 228362314 | rs4846914 | A | G | 39.5% | 0.059 | 0.953 | 1.006 | 0.814 - 1.243 | ^{abcdef} |

| | | | | | | | | | | | |
|---|---|---|---|---|---|---|---|---|---|---|---|
| No consistent trend towards HUL raising or lowering alleles conferring risk of AMD was seen, suggesting that the LIPC No consistent trend towards HDL raising or lowering alleles conferring risk of AMD was seen, suggesting that the LIPC association may be a consequence of pleiotropy. Chr: chromosome; Position: base pair position in NCBI 36.1; SNP: single nucleotide polymorphism; HLD-raising allele: allele reported in Kathiresan et al (25) as the allele responsible for raising HDL; Minor Allele: SNP minor allele; MAF: Minor Allele Frequency; Z-score: weighted average and direction of minor allele signal; Meta *P*: *P*-value for the association between the minor allele and AMD; OR: odds ratio for the minor allele; 95% CI: confidence interval for the OR. The replication cohorts are (a) Tufts/MGH GWAS, (b) Tufts/MGH replication, (c) JHU, (d) COL, (e) UT, (f) FR-CRET, and (g) WASH-U. | | | | | | | | | | | |

### Example 2.

### LIPC Gene Variant, rs10468017, and Smoking, Body Mass Index, Dietary Lutein Associations with Advanced Age-Related Macular Degeneration.

### ABSTRACT

**Objectives:** Recently a novel locus in the *LIPC* gene was found to be significantly related to advanced forms of AMD in our large genome-wide association study. We evaluated the association of this gene with previously identified genetic variants and with modifiable lifestyle factors associated with AMD.
**Methods:** Caucasian participants in the Age-Related Eye Disease Study with advanced AMD (n= 545 cases) or no AMD (n=275 controls) were evaluated. AMD status was determined by grading of fundus photographs. Covariates included know covariates for AMD: cigarette smoking, body mass index (BMI), and dietary lutein. Individuals were genotyped for the novel rs10468017 variant in the *LIPC* gene as well as seven previously identified AMD genetic loci. Unconditional logistic regression analyses were performed.
**Results:** The T allele of the *LIPC* variant was associated with a reduced risk of advanced AMD, with odds ratios (OR) of 0.50 (95% confidence interval (CI) 0.20-0.90, P= .014) for the TT genotype versus the CC genotype, controlling for age, gender, smoking, body mass index, and nutritional factors. Controlling for seven other AMD genetic variants did not materially change the association (OR 0.50, 95% CI 0.20-1.1, P = .077). The magnitude of the effect was similar for both the atrophic and neovascular types of advanced AMD. Cigarette smoking and higher body mass index increased risk, and higher intake of dietary lutein reduced risk of advanced AMD, adjusting for all eight genetic variants. *LIPC* was not associated with the other AMD genes. There were no interactions with smoking or BMI, and there was a non-significant trend toward an interaction between lutein intake and the TT genotype of *LIPC.*
**Conclusions:** Adjusting for demographic and lifestyle factors and seven other genetic loci, the T allele of the *LIPC* variant reduced risk of both geographic atrophy and neovascular forms of advanced AMD. Lifestyle factors modified risk of AMD within most *LIPC* genotype groups.

The US twin study of age-related macular degeneration (AMD) quantified the proportions of variance in early, intermediate, and advanced forms of this disease due to genetic and environmental factors as 46 to 71% and 19-37%, respectively. Several environmental factors have been identified including cigarette smoking, higher body mass index (BMI), and nutritional factors. A genetic effect was suggested for several years based on clinical observations, familial aggregation and linkage studies, and has been confirmed by studies showing associations between AMD and several genetic loci.

We conducted a large genome-wide association study (GWAS) of 979 cases of advanced AMD and 1709 controls, with replication of our top results in independent cohorts with a total of 4337 cases and 2077 controls. Our scan identified the hepatic lipase gene (*LIPC*) in the high-density lipoprotein cholesterol (HDL-c) pathway as a novel locus for AMD risk, with a protective effect for the minor T allele. Hepatic lipase is a form of lipase. It is expressed in the liver and adrenal glands. One of the principal functions of hepatic lipase is to convert IDL to LDL. *LIPC* encodes hepatic triglyceride lipase, which is expressed in the liver. *LIPC* has the dual functions of triglyceride hydrolase and ligand/bridging factor for receptor-mediated lipoprotein uptake. A separate GWAS independently corroborated the *LIPC* association with AMD but that finding did not reach the level of genome-wide significance.

We further explored this locus and found that the association was strongest at the functional variant in the promoter region (single nucleotide polymorphism (SNP) rs10468017), which influences LIPC expression. The goals of this study were to evaluate the association between this new genetic variant and advanced AMD, the relationship between this gene and the two distinct advanced "dry and wet" phenotypes, and to assess the *LIPC* gene-environment associations and interactions with demographic, personal and lifestyle factors.

### METHODS

The Age-Related Eye Disease Study (AREDS) included a randomized clinical trial to assess the effect of antioxidant and mineral supplements on risk of AMD and cataract, and a longitudinal study of progression of AMD which ended in December, 2005. Study procedures have been previously reported. Based on ocular examination and reading center photographic grading of fundus photographs, Caucasian participants in this study were divided into two main groups representing the most discordant phenotypes: no AMD defined as either no drusen or nonextensive small drusen (n=268), or advanced AMD with visual loss (n=532). The advanced form of AMD was then reclassified into the two subtypes of either non-central or central geographic atrophy (GA, n=138) or neovascular disease (NV, n=394), independent of visual acuity level, using the Clinical Age-Related Maculopathy Grading System, to determine whether results differed between the two advanced AMD phenotypes. Risk factor data was obtained at the baseline visit from questionnaires and height and weight measurements. DNA samples were obtained from the AREDS Genetic Repository. The single nucleotide polymorphism, rs10468017, a functional variant of the *LIPC* gene on chromosome 15q22 was assessed. In addition, variants in seven known AMD genes were also determined : 1)the common single nucleotide polymorphism (SNP) in exon 9 of the CFH gene on chromosome 1q31 (rs 1061170), a change 1277T>C, resulting in a substitution of histidine for tyrosine at codon 402 of the CFH protein,Y402H, 2) *CFH* rs 1410996, an independently associated single nucleotide polymorphism (SNP) variant within intron 14 of *CFH,* 3) SNP rs10490924 in the *ARMS2*/*HTRA1* region of chromosome 10, a non-synonymous coding SNP variant in exon 1, resulting in a substitution of the amino acid serine for alanine at codon 69, 4) Complement component 2 or *C2 E318D* (rs9332739), the non-synonymous coding SNP variant in exon 7 of *C2* resulting in the amino acid glutamic acid changing to aspartic acid at codon 318, 5) Complement Factor B or *CFB R32Q* (rs641153), the non-synonymous coding SNP variant in exon 2 of *CFB* resulting in the amino acid glutamine changing to arginine at codon 32, 6) Complement component 3 or *C3 R102G* (rs2230199), the non-synonymous coding SNP variant in exon 3 of *C3* resulting in the amino acid glycine to arginine at codon 102, on chromosome 19, and 7) Complement Factor I or *CFI* (rs10033900) on chromosome 4. For the genetic variant on chromosome 10, *ARMS2*/*HTRA1,* it remains a subject of debate whether the gene *HTRA1* adjacent to it may in fact be the AMD-susceptibility gene on 10q26; however, the relevant SNPs in these 2 genes have been reported to be nearly perfectly correlated. Thus, while the other snp is a promising candidate variant, rs 10490924 used in this study can be considered a surrogate for the causal variant which resides in this region. For the *C2*/*CFB* genes, there are two independent associations to the *C2*/*CFB* locus, but because of linkage disequilibrium we do not know which of the two genes or both are functionally affected. Genotyping was performed using primer mass extension and MALDI-TOF MS analysis by the MassEXTEND methodology of Sequenom (San Diego, CA) at the Broad Institute Center for Genotyping and Analysis, Cambridge, MA.

### Statistical Analyses

Individuals with advanced AMD, as well as the GA and NV subtypes, were compared to the control group of persons with no AMD, with regard to genotype and risk factor data. Multivariate unconditional logistic regression analyses were performed to evaluate the relationships between AMD and *LIPC,* controlling for age (70 or older, younger than 70); gender; education (high school or less, more than high school); cigarette smoking (never, past, current); BMI which was calculated as the weight in kilograms divided by the square of the height in meters (< 25, 25-29.9, and ≥30); dietary lutein (micrograms) which was determined from food frequency questionnaires; and assignment to a supplement containing antioxidants or a supplement not containing antioxidants. Dietary lutein was included in the models since it is related to AMD ( Seddon et al JAMA 1994 and AREDS 2009), and also since HDL is the major lipoprotein transporter of lutein and zeaxanthin. Changes in the efficiency of carotenoid delivery is one mechanism by which *LIPC* genetic variation could be related to AMD.

A separate statistical model including all of the above factors plus the seven other genetic variants was also evaluated. The association between the *LIPC* gene and these variants was assessed. Tests for multiplicative interactions between genes and also between genes and environmental factors were performed using cross product terms according to genotype and the individual risk factors. Odds ratios and 95% confidence intervals were calculated for each risk factor and within the genotype groups.

### RESULTS

The distribution of the demographic, personal, and lifestyle variables according to the *LIPC* genotypes for cases and controls with geographic atrophy and neovascular disease are shown in **Table 16.** There were no significant differences in age, gender, education, smoking, BMI, AREDS treatment, or calorie- adjusted lutein among the *LIPC* genotypes.
**Table 17** displays the association between the *LIPC* gene and other known AMD genetic loci. There were no significant gene-gene interactions seen between this gene and the other loci.
Table 18 shows the odds ratios for the multivariate models, comparing all advanced AMD cases, as well as GA and NV cases, with controls, for the *LIPC* variant, while adjusting for demographic and behavioral risk factors. Controlling for age, gender, education, smoking, body mass index, AREDS treatment, and dietary lutein in multivariate model 1, advanced cases were less likely to have the T allele, with OR of 0.50 (95% confidence interval (CI) 0.2-0.9) for advanced AMD, p trend=0.047. Addition of the other seven genotypes (multivariate model 2), did not materially alter the effect of this new genetic variant (OR 0.50, 95% CI 0.2-1.1). There were minimal differences between GA and NV for this locus. For GA in model 1, the OR was 0.50 (95% CI 0.2-1.3) for the TT genotype, and for NV the OR was 0.40 (95% CI 0.2-0.9). **Table 18** also shows the associations between advanced AMD, GA, and NV with older age, less education, past and current smoking, high BMI, and lower levels of lutein, compared with controls.

Cigarette smoking was associated with a statistically significant increased risk of advanced AMD for both subtypes, controlling for genotype and other factors, with MV1 OR's for current smoking ranging from 3.9-4.0 and 1.5-1.8 for past smoking. Body mass index of 30 kg/m² or higher increased risk for advanced AMD and this elevated risk was slightly higher among neovascular cases, (OR 2.1,95% CI 1.3-3.4), compared with geographic atrophy (OR 1.8, 95% CI 01.0-3.2), although this small difference in OR's between the two advanced forms of AMD was not statistically significant. Higher lutein intake tended to reduce risk of AMD, with OR 0.6 (95% CI 0.4-1.0) for the third quartile vs. the first for overall advanced AMD. Additional adjustment for the other seven genes (multivariate 2 models) did not materially alter these associations. There were no substantial differences between GA and NV in these analyses of the covariates.

We assessed the effect of interactions between LIPC genotypes and lifestyle factors on risk of AMD and these results are shown in **Table 19.** Higher BMI and smoking, as well as lower lutein intake tended to increase risk of AMD overall. None of the interactions had a significant effect on risk of AMD. However, there was a trend toward an interaction between lutein intake and the TT genotype, with a slightly stronger association between higher lutein intake and lower risk of GA within the TT genotype than the CC genotype. Due to the small number of subjects in this subset, however, confirmation is required in larger studies which have information about diet and all the other covariates.

### DISCUSSION

To our knowledge, this is the first evaluation of the relationship between the *LIPC* functional variant and advanced AMD, controlling for demographic and behavioral factors including BMI, smoking, and dietary factors, as well as all known AMD genes. *LIPC* and environmental factors were independently associated with advanced AMD, the leading cause of visual impairment and vision-related reduced quality of life among elderly individuals. After adjustment for demographic and behavioral factors, the newly discovered variant of the *LIPC* gene located on chromosome 15q22, rs10468017, was strongly associated with advanced AMD. A small non-significant difference in effect was seen between GA and NV, although this could be due to the smaller sample size of the GA group. After controlling for *LIPC* genotype, the modifiable lifestyle factors, higher BMI, smoking, and dietary lutein were statistically significantly related to increased risk of advanced AMD. Similar to our previous findings with other genetic variants, there was no interaction between smoking and genotype.

(Seddon-Hum Hered paper 2006, Seddon JAMA 2007 Progression paper, IOVS Seddon prediction model paper 2009) However, there was a non-significant trend for a slightly stronger association between higher lutein intake and lower risk of GA within the TT genotype than the CC genotype. There were no interactions between *LIPC* and the other known AMD genes.

The association between *LIPC* polymorphisms and AMD is biologically plausible, because this gene is involved with the HDL cholesterol pathway, and cardiovascular risk factors are associated with AMD. These data also support our previous findings and that of others which show increased risk of AMD with smoking (Seddon JAMA 1996) and higher BMI (Seddon Arch Ophthalmol 2003), and reduced risk with higher lutein intake (Seddon JAMA 1994). The new findings in this report are that these behaviors make a difference even with genetic predisposing factors including *LIPC.*

Strengths of the study include the large, well characterized population of Caucasian patients with and without advanced AMD from various geographic regions around the US, standardized collection of risk factor information, direct measurements of height and weight, and classification of maculopathy by ophthalmologic examinations and fundus photography. Misclassification was unlikely since grades were assigned without knowledge of risk factors or genotype. We controlled for known AMD risk factors, including age and education, as well as antioxidant status, in the assessment of BMI, smoking, dietary lutein, and genotype.

Both the environmental and genetic risk factors were independently associated with AMD, when considered simultaneously, after adjustment for these factors. There may be some other unmeasured factors that might still be confounding these relationships, but they would have to be highly related to genotype, smoking and BMI, and a strong risk factor for AMD to explain these results. Although this is a selected population, cases likely represent the typical patient with AMD, and the overall population is similar to others in this age range in terms of smoking and prevalence of obesity, as well as the distribution of the *LIPC* genotype. Furthermore, the biological effects of LIPC and the modifiable factors are not likely to differ in major ways among various Caucasian populations with AMD. Prospective studies are also needed.

| Table 16: Distribution of Demographic and Behavioral Risk Factors for Advanced Age-Related Macular Degeneration According to LIPC Genotypes | | | | | | | | | | | | | | | | | | | | | | |
|---|---|---|---|---|---|---|---|---|---|---|---|---|---|---|---|---|---|---|---|---|---|---|
| | | | | | | | | | | | | | | | | | | | | | | |

| | | | | | Controls | | | | | Geographic | | Atrophy | | | | | | | Neovascular Disease | | | |
|---|---|---|---|---|---|---|---|---|---|---|---|---|---|---|---|---|---|---|---|---|---|---|
| | | | CC | | CT | | TT | P-Value* | | CC | | CT | | TT | P-Value | | CC | | CT | | TT | P-Value |
| | | N | % | N | % | N | % | | N | % | N | % | N | % | | N | % | N | % | N | % | |
| | | | | | | | | | | | | | | | | | | | | | | |
| Baseline Age | | | | | | | | | | | | | | | | | | | | | | |
| 50-69 | | 89 | (70) | 92 | (76) | 21 | (81) | | 38 | (46) | 28 | (53) | 4 | (57) | | 102 | (48) | 89 | (51) | 4 | (19) | |
| 70-95 | | 39 | (31) | 29 | (24) | 5 | (19) | 0.30 | 44 | (54) | 22 | (44) | 3 | (43) | 0.57 | 109 | (52) | 85 | (49) | 17 | (81) | 0.01 |
| | | | | | | | | | | | | | | | | | | | | | | |
| Gender | | | | | | | | | | | | | | | | | | | | | | |
| Male | | 58 | (45) | 57 | (47) | 10 | (39) | | 44 | (54) | 24 | (48) | 3 | (43) | | 88 | (42) | 64 | (37) | 6 | (29) | |
| Female | | 70 | (55) | 64 | (53) | 16 | (62) | 0.49 | 38 | (46) | 26 | (52) | 4 | (57) | 0.58 | 123 | (58) | 110 | (63) | 15 | (71) | 0.24 |
| | | | | | | | | | | | | | | | | | | | | | | |
| Education | | | | | | | | | | | | | | | | | | | | | | |
| High School or Less | | 7 | (6) | 4 | (3) | 1 | (4) | | 7 | (9) | 6 | (12) | 0 | 0.0 | | 32 | (15) | 11 | (6) | 5 | (24) | |
| College or more | | 121 | (95) | 117 | (97) | 25 | (96) | 0.80 | 75 | (92) | 44 | (88) | 7 | 100.0 | 0.47 | 179 | (85) | 163 | (94) | 16 | (76) | 0.24 |
| | | | | | | | | | | | | | | | | | | | | | | |
| Smoking | | | | | | | | | | | | | | | | | | | | | | |
| Never | | 67 | (52) | 56 | (46) | 15 | (58) | | 30 | (37) | 16 | (32) | 3 | (43) | | 78 | (37) | 70 | (40) | 11 | (52) | |
| Former | | 58 | (45) | 57 | (47) | 10 | (39) | 0.50 | 42 | (51) | 31 | (62) | 3 | (43) | 0.71 | 108 | (51) | 82 | (47) | 8 | (38) | 0.18 |
| Current | | 3 | (2) | 8 | (7) | 1 | (4) | 0.99 | 10 | (12) | 3 | (6) | 1 | (14) | 1.00 | 25 | (12) | 22 | (13) | 2 | (10) | 0.48 |
| | | | | | | | | | | | | | | | | | | | | | | |
| BMI | | | | | | | | | | | | | | | | | | | | | | |
| <25.0 | | 39 | (31) | 40 | (33) | 9 | (35) | 0.40 | 23 | (28) | 16 | (32) | 3 | (43) | 0.96 | 45 | (21) | 51 | (29) | 4 | (19) | 0.80 |
| 25.0-29.9 | | 55 | (43) | 59 | (49) | 13 | 50.0 | 0.98 | 35 | (43) | 20 | (40) | 1 | (14) | 0.17 | 100 | (47) | 74 | (43) | 10 | (48) | 0.86 |
| ≥30.0 | | 34 | (27) | 22 | (18) | 4 | (15) | 0.35 | 24 | (29) | 14 | (28) | 3 | (43) | 0.97 | 66 | (31) | 49 | (28) | 7 | (33) | 0.79 |
| | | | | | | | | | | | | | | | | | | | | | | |
| Supplements | | | | | | | | | | | | | | | | | | | | | | |
| No Antioxidants | | 69 | (54) | 66 | (55) | 17 | (65) | | 44 | (54) | 30 | (60) | 4 | (57) | | 109 | (52) | 84 | (48) | 8 | (38) | |
| Amioxidants | | 59 | (46) | 55 | (46) | 9 | (35) | 0.28 | 38 | (46) | 20 | (40) | 3 | (43) | 0.84 | 102 | (48) | 90 | (52) | 13 | (62) | 0.24 |
| | | | | | | | | | | | | | | | | | | | | | | |
| Lutein:Calorie Adjusted, | | | | | | | | | | | | | | | | | | | | | | |
| Sex specific (mean µg) | | | 1501.6 | | 1520.3 | | 1573.7 | 0.68 | | 1278.1 | | 1448.8 | | 1152.7 | 0.57 | | 1325.2 | | 1351.7 | | 1309.8 | 0.85 |
| | | | | | | | | | | | | | | | | | | | | | | |
| *P-Value indicates the relationship between the number of T alleles for LIPC and each category of the variable. | | | | | | | | | | | | | | | | | | | | | | |

| Table 17: Associations Between LIPC Genotypes and Other Genetic Variants Related to Age-Related Macular Degeneration | | | | | | | | | | | | | | | | | | | | | | |
|---|---|---|---|---|---|---|---|---|---|---|---|---|---|---|---|---|---|---|---|---|---|---|
| | | | | | | | | | | | | | | | | | | | | | | |

| | | | | | Grade 1 | | | | | | | Grade 4 | | | | | | | Grade 5 | | | |
|---|---|---|---|---|---|---|---|---|---|---|---|---|---|---|---|---|---|---|---|---|---|---|
| | | | LIPC:rs10468017 | | | | | | | | LIP:Crs10468017 | | | | | | | LIP::rs10468017 | | | | |
| | | | CC | | CT | | TT | P-Value* | | CC | | CT | | TT | P-Value* | | CC | | CT | | TT | P-Value* |
| | | N | % | N | % | N | % | | N | % | N | % | N | % | | N | % | N | % | N | % | |
| | | | | | | | | | | | | | | | | | | | | | | |

| CFH:rs1061170 (Y402H) | | | | | | | | | | | | | | | | | | | | | | |
|---|---|---|---|---|---|---|---|---|---|---|---|---|---|---|---|---|---|---|---|---|---|---|
| TT | | 42 | (33) | 57 | (47) | 14 | (54) | | 14 | (17) | 8 | (16) | 1 | (14) | | 37 | (18) | 24 | (14) | 4 | (19) | |
| CT | | 67 | (52) | 48 | (40) | 8 | (31) | | 32 | (39) | 20 | (40) | 3 | (43) | | 98 | (47) | 77 | (44) | 9 | (43) | |
| CC | | 19 | (15) | 16 | (13) | 4 | (15) | 0.096 | 36 | (44) | 22 | (44) | 3 | (43) | 0.86 | 76 | (36) | 73 | (42) | 8 | (38) | 0.88 |
| | | | | | | | | | | | | | | | | | | | | | | |

| CFH:rs1410996 | | | | | | | | | | | | | | | | | | | | | | |
|---|---|---|---|---|---|---|---|---|---|---|---|---|---|---|---|---|---|---|---|---|---|---|
| TT | | 16 | (13) | 25 | (21) | 6 | (23) | | 1 | (1) | 2 | (4) | 1 | (14) | | 10 | (5) | 5 | (3) | 1 | (5) | |
| CT | | 65 | (51) | 64 | (53) | 14 | (54) | | 27 | (33) | 11 | (22) | 3 | (43) | | 62 | (29) | 44 | (25) | 6 | (29) | |
| CC | | 47 | (37) | 32 | (27) | 6 | (23) | 0.23 | 54 | (66) | 37 | (74) | 3 | (43) | 0.035 | 139 | (66) | 125 | (72) | 14 | (67) | 0.99 |

| ARMS2/HTRA1 rs10490924 | | | | | | | | | | | | | | | | | | | | | | |
|---|---|---|---|---|---|---|---|---|---|---|---|---|---|---|---|---|---|---|---|---|---|---|
| GG | | I 89 | (70) | 78 | (65) | 16 | (62) | | 27 | (33) | 21 | (42) | 3 | (43) | | 62 | (29) | 48 | (28) | 6 | (29) | |
| GT | | 34 | (28) | 39 | (32) | 10 | (39) | | 40 | (49) | 24 | (48) | 4 | (57) | | 108 | (51) | 83 | (48) | 8 | (38) | |
| TT | | 3 | (2) | 4 | (3) | 0 | 0.0 | 0.60 | 15 | (18) | 5 | (10) | 0 | 0.0 | 0.18 | 41 | (19) | 43 | (25) | 7 | (33) | 0.16 |
| | | | | | | | | | | | | | | | | | | | | | | |

| CFB:rs641153 (R32Q) | | | | | | | | | | | | | | | | | | | | | | |
|---|---|---|---|---|---|---|---|---|---|---|---|---|---|---|---|---|---|---|---|---|---|---|
| CC | | 101 | (79) | 91 | (75) | 19 | (73) | | 76 | (93) | 47 | (94) | 7 | 100.0 | | 191 | (91) | 162 | (93) | 20 | (95) | |
| CT/TT | | 27 | (21) | 30 | (25) | 7 | (27) | 0.57 | 6 | (7) | 3 | (6) | 0 | 0.0 | 0.45 | 20 | (10) | 12 | (7) | 1 | (5) | 0.45 |
| | | | | | | | | | | | | | | | | | | | | | | |

| C2:rs9332739 (E318D) | | | | | | | | | | | | | | | | | | | | | | |
|---|---|---|---|---|---|---|---|---|---|---|---|---|---|---|---|---|---|---|---|---|---|---|
| GG | | 116 | (91) | 106 | (88) | 24 | (92) | | 79 | (96) | 49 | (98) | 7 | 100.0 | | 197 | (93) | 167 | (96) | 21 | 100.0 | |
| CC/CG | | 12 | (9) | 15 | (12) | 2 | (8) | 0.71 | 3 | (4) | 1 | (2) | 0 | 0.0 | 0.57 | 14 | (7) | 7 | (4) | 0 | 0.0 | 0.19 |
| | | | | | | | | | | | | | | | | | | | | | | |

| C3:rs2230199(R102h) | | | | | | | | | | | | | | | | | | | | | | |
|---|---|---|---|---|---|---|---|---|---|---|---|---|---|---|---|---|---|---|---|---|---|---|
| CC | | 82 | (64) | 74 | (61) | 14 | (54) | | 42 | (51) | 25 | (50) | 4 | (57) | | 98 | (47) | 88 | (51) | 1u | (48) | |
| CG | | 44 | (34) | 40 | (33) | 11 | (42) | | 33 | (40) | 23 | (46) | 2 | (29) | | 95 | (45) | 72 | (41) | 10 u | (48) | |
| GG | | 2 | (2) | 7 | (6) | 1 | (4) | 0.60 | 7 | (9) | 2 | (4) | 1 | (14) | 0.66 | 18 | | 14 | (8) | 1 | (5) | 0.56 |
| | | | | | | | | | | | | | | | | | | | | | | |

| CFI:rs10033900 | | | | | | | | | | | | | | | | | | | | | | |
|---|---|---|---|---|---|---|---|---|---|---|---|---|---|---|---|---|---|---|---|---|---|---|
| CC | | 36 | (28) | 40 | (33) | 11 | (42) | | 18 | (22) | 7 | (14) | 1 | (14) | | 47 | (22) | 41 | (24) | 6 | (29) | |
| CT | | 59 | (46) | 64 | (53) | 11 | (42) | | 40 | (49) | 28 | (56) | 4 | (57) | | 118 | (56) | 76 | (44) | 12 | (57) | |
| TT | | 33 | (26) | 17 | (14) | 4 | (15) | 0.30 | 24 | (29) | 15 | (30) | 2 | (29) | 0.99 | 46 | (22) | 57 | (33) | 3 | (14) | 0.44 |
| | | | | | | | | | | | | | | | | | | | | | | |
| *P for trend for overall association between number of T aileles for LIPC and number of risk/protective alleles | | | | | | | | | | | | | | | | | | | | | | |
| for other genotypes, or presence of at least 1 risk/protective allele for other genotypes | | | | | | | | | | | | | | | | | | | | | | |

| **Table 18.** | Multivariate Analyses of Associations Between Advanced Age-Related Macular Degeneration (AMD), *LIPC* Genotype, | | | | | | | | | | |
|---|---|---|---|---|---|---|---|---|---|---|---|
| | and Demographic, Genetic, and Behavioral Risk Factors | | | | | | | | | | |
| | | Advanced AMD | | | Geographic Atrophy | | | Neovascular AMD | | | |
| | | | | | | | | | | | |
| No. of cases/controls | | 545/275 | | | 139/275 | | | 406/275 | | | |
| | | | | | | | | | | | |
| | | OR (CI) | P Value | | OR (CI) | P Value | | OR (CI) | P Value | | |
| | | | | | | | | | | | |
| | | | | | | | | | | | |
| *LIPC* Genotype | | | | | | | | | | | |
| Multivariate¹ | | | | | | | | | | | |
| C C | | 1.0 | | | 1.0 | | | 1.0 | | | |
| C T | | 0.9 (0.7 - 1.2) | 0.53 | | 0.7 (0.4 - 1.1) | 0.14 | | 1.0 (0.7 - 1.4) | 0.87 | | |
| T T | | 0.5 (0.2 - 0.9) | 0.014 | | 0.5 (0.2 - 1.3) | 0.15 | | 0.4 (0.2 - 0.9) | 0.02 | | |
| Number of T alleles | | | 0.047 | (p - trend) | | 0.062 | (p - trend) | | 0.11 | (p - trend) | |
| | | | | | | | | | | | |
| Multivariate² | | | | | | | | | | | |
| C C | | 1.0 | | | 1.0 | | | 1.0 | | | |
| C T | | 1.0 (0.6 - 1.4) | 0.85 | | 0.9 (0.5 - 1.6) | 0.81 | | 1.0( 0.6 - 1.5) | 0.94 | | |
| T T | | 0.5 (0.2 - 1.1) | 0.077 | | 0.7 (0.2 - 2.2) | 0.56 | | 0.4 (0.2 - 1.1) | 0.066 | | |
| | | | 0.21 | (p - trend) | | 0.59 | (p - trend) | | 0.22 | (p - trend) | |
| Number of T alleles | | | | | | | | | | | |
| Age | | | | | | | | | | | |
| Multivariate¹ | | | | | | | | | | | |
| <70 | | 1.0 | | | 1.0 | | | 1.0 | | | |
| ≥ 70 | | 3.2 (2.3 - 4.4) | <0.0001 | | 2.9 (1.8 - 4.6) | <0.0001 | | 3.3 (2.3 - 4.7) | <0.0001 | | |
| | | | | | | | | | | | |
| Multivariate² | | | | | | | | | | | |
| <70 | | 1.0 | | | 1.0 | | | 1.0 | | | |
| ≥70 | | 3.3 (2.2 - 4.9) | <0.0001 | | 3.1 (1.8 - 5.4) | <0.0001 | | 3.9 (2.5 - 6.0) | <0.0001 | | |
| | | | | | | | | | | | |
| | | | | | | | | | | | |
| Gender | | | | | | | | | | | |
| Multivariate¹ | | | | | | | | | | | |
| Male | | 1.0 | | | 1.0 | | | 1.0 | | | |
| Female | | 0.8 (0.6 - 1.1) | 0.13 | | 1.2 (0.8 - 1.9) | 0.44 | | 0.7 (0.5 - 1.0) | 0.026 | | |
| | | | | | | | | | | | |
| Multivariate² | | | | | | | | | | | |
| Male | | 1.0 | | | 1.0 | | | 1.0 | | | |
| Female | | 0.9 (0.6 - 1.3) | 0.42 | | 1.2 (0.7 - 2.1) | 0.44 | | 0.7 (0.5 - 1.1) | 0.13 | | |
| | | | | | | | | | | | |
| | | | | | | | | | | | |
| Education | | | | | | | | | | | |
| Multivariate¹ | | | | | | | | | | | |
| ≤ HS | | 1.0 | | | 1.0 | | | 1.0 | | | |
| > HS | | 0.4 (0.2 - 0.8) | 0.007 | | 0.4 (0.2 - 1.0) | 0.061 | | 0.4 (0.2 - 0.7) | 0.005 | | |
| | | | | | | | | | | | |
| Multivariate² | | | | | | | | | | | |
| ≤ HS | | 1.0 | | | 1.0 | | | 1.0 | | | |
| > HS | | 0.5 (0.2 - 1.1) | 0.079 | | 0.4 (0.1 - 1.1) | 0.074 | | 0.6 (0.2 - 1.4) | 0.20 | | |
| | | | | | | | | | | | |
| | | | | | | | | | | | |
| Smoking | | | | | | | | | | | |
| Multivariate¹ | | | | | | | | | | | |
| Never | | 1.0 | | | 1.0 | | | 1.0 | | | |
| Past | | 1.5 (1.1 - 2.2) | 0.010 | | 1.8 (1.1 - 2.9) | 0.016 | | 1.5 (1.1 - 2.1) | 0.024 | | |
| Current | | 3.9 (2.0 - 7.7) | <0.0001 | | 4.0 (1.6 - 9.6) | 0.002 | | 3.9 (1.9 - 7.9) | 0.0002 | | |
| | | | <0.0001 | (p - trend) | | 0.001 | (p - trend) | | <0.0001 | (p - trend) | |
| | | | | | | | | | | | |
| Multivariate² | | | | | | | | | | | |
| Never | | 1.0 | | | 1.0 | | | 1.0 | | | |
| Past | | 2.0 (1.3 - 3.0) | 0.001 | | 1.8 (1.0 - 3.1) | 10.054 | | 2.1 (1.3 - 3.3) | 0.001 | | |
| Current | | 4.5 (2.1 - 10.1) | 0.0002 | | 4.3 (1.4 - 13.0) | 0.009 | | 4.8 (2.1 - 11.2) | 0.0002 | | |
| | | | <0.0001 | (p - trend) | | 0.005 | (p - trend) | | <0.0001 | (p - trend) | |
| | | | | | | | | | | | |
| | | | | | | | | | | | |
| BMI | | | | | | | | | | | |
| Multivariate¹ | | | | | | | | | | | |
| <25 | | 1.0 | | | 1.0 | | | 1.0 | | | |
| 25-29.9 | | 1.3 (0.9 - 1.9) | 0.15 | | 1.1 (0.6 - 1.8) | 0.85 | | 1.4 (1.0 - 2.1) | 0.085 | | |
| ≥30 | | 2.0 (1.3 - 3.1) | 0.002 | | 1.8 (1.0 - 3.2) | 0.057 | | 2.1 (1.3 - 3.4) | 0.002 | | |
| | | | 0.002 | (p - trend) | | 0.064 | (p - trend) | | 0.002 | (p - trend) | |
| | | | | | | | | | | | |
| Multivariate² | | | | | | | | | | | |
| <25 | | 1.0 | | | 1.0 | | | 1.0 | | | |
| 25-29.9 | | 1.2 (0.7 - 1.9) | 0.50 | | 0.9 (0.5 - 1.7) | 0.72 | | 1.4 (0.8 - 2.3) | 0.20 | | |
| ≥30 | | 2.0 (1.2 - 3.3) | 0.010 | | 1.7 (0.8 - 3.5) | 0.15 | | 2.3 (1.3 - 4.0) | 0.005 | | |
| | | | 0.010 | (p - trend) | | 0.18 | (p - trend) | | 0.005 | (p - trend) | |
| | | | | | | | | | | | |
| | | | | | | | | | | | |
| Calorie adjusted, sex-specific Lutein | | | | | | | | | | | |
| Multivariate¹ | | | | | | | | | | | |
| 1 | | 1.0 | | | 1.0 | | | 1.0 | | | |
| 2 | | 0.7 (0.5 - 1.1) | 0.13 | | 0.7 (0.4 - 1.3) | 0.25 | | 0.8 (0.5 - 1.2) | 0.27 | | |
| 3 | | 0.6 (0.4 - 1.0) | 0.029 | | 0.6 (0.4 - 1.1) | 0.11 | | 0.7 (0.4 - 1.0) | 0.056 | | |
| | | | 0.031 | (p - trend) | | 0.11 | (p-trend) | | 0.057 | (p - trend) | |
| | | | | | | | | | | | |
| Multivariate² | | | | | | | | | | | |
| 1 | | 1.0 | | | 1.0 | | | 1.0 | | | |
| 2 | | 0.8 (0.5 - 1.3) | 0.46 | | 0.7 (0.4 - 1.3) | 0.28 | | 0.9 (0.6 - 1.6) | 0.79 | | |
| 3 | | 0.7 (0.4 - 1.1) | 0.099 | | 0.6 (0.3 - 1.1) | 0.077 | | 0.8 (0.5 - 1.3) | 0.30 | | |
| | | | 0.097 | (p - trend) | | 0.078 | (p - trend) | | 0.29 | (p - trend) | |
| | | | | | | | | | | | |
| | | | | | | | | | | | |
| OR= Odds Ratio, GI= 95% Confidence Interval | | | | | | | | | | | |
| Multivariate 1= Model Adjusted for age (50 - 69, 70 -95), gender, education (≤ high school vs. > high school), smoking (never, past, current), | | | | | | | | | | | |
| BMI (<25, 25 - 29.9, ≥30), *LIPC* (CC, CT, TT), antioxidant treatment (supplement containing antioxidants vs. supplement containing | | | | | | | | | | | |
| no antioxidants), calorie adjusted lutein | | | | | | | | | | | |
| | | | | | | | | | | | |
| Multivariate 2 = Model Adjusted for all variables in Multivariate 1 p us CFB (CC, CT/TT), *CFH* Y402H (TT, CT, CC), CFHrs1410966 (TT, CT, CC), | | | | | | | | | | | |
| C2 (GG, *CG*/*CC*), *ARMS2*/*HTRA1*(GG, GT, TT), C3 (CC, CG, GG), *CFI* (CC, CT, TT). | | | | | | | | ' | | I | |

| Table 19. Assessment of Effect of Interactions Between *LIPC* Genotype and Lifestyle Factors on Risk of Age-Related | | | | | | | | | |
|---|---|---|---|---|---|---|---|---|---|
| Macular Degeneration (AMD) | | | | | | | | | |
| | | | | | *LIPC* Genotype | | | | P (Trend) |
| | | | CC | | CT | | TT | | for No. of |
| | | | | | | | | | C alleles |
| No. of cases | | | | | | | | | |
| | Advanced AMD | | 293 | | 224 | | 28 | | |
| | Geographic Atrophy | | 82 | | 50 | | 7 | | |
| | Neovascular AMD | | 211 | | 174 | | 21 | | |
| | | | | | | | | | |
| No. of controls | | | 128 | | 121 | | 26 | | |
| | | | | | | | | | |
| | | | OR (CI) | | OR (CI) | | OR (CI) | | |
| BMI | Advanced AMD | | | | | | | | |
| | <25 | | 1.0 | | 1.1(0.6-2.0) | | 0.6 l (0.2-1.8) | | |
| | 25+ | | 1.7(1.0-2.8) | | 1.4 (0.8-2.3) | | 0.7(0.3-1.6) | | |
| | | | | | | | | | |
| | P (Interaction) | | | | 0.40 (CT vs. CC) | | 0.59 (TT vs. CC0 | | 0.40 |
| | | | | | | | | | |
| | Geographic Atrophy | | | | | | | | |
| | <25 | | 1.0 | | 0.7(0.3-1.7) | | 0.7(0.2-2.9) | | |
| | 25+ | | 1.3(0.7-2.6) | | 0.9(0.4-1.8) | | 0.6(0.2-2.0) | | |
| | P (Interaction) | | | | 0.82 (CT vs. CC) | | 0.63 (TT vs. CC) | | 0.64 |
| | | | | | | | | | |
| | Neovascular AMD | | | | | | | | |
| | <25 | | 1.0 | | 1.2 (0.7-2.3) | | 0.5(0.1-1.9) | | |
| | 25+ | | 1.8 (1.1-3.1) | | 1.6(0.9-2.8) | | 0.8(0.3-1.8) | | |
| | | | | | | | | | |
| | P (Interaction) | | | | 0.40 (CT vs. CC) | | 0.82 (TT vs. CC) | | 0.48 |
| | | | | | | | | | |
| | | | | | | | | | |
| Smoking | Advanced AMD | | | | | | | | |
| | Never | | 1.0 | | 1.1(0.7-1.8) | | 0.6(0.2-1.3) | | |
| | Ever | | 2.1 (1.4-3.3) | | 1.6(1.0-2.5) | | 0.7(0.3-1.8) | | |
| | | | | | | | | | |
| | P (Interaction) | | | | 0.27 (CT vs CC) | | 0.43 (TT vs. CC) | | 0.30 |
| | | | | | | | | | |
| | Geographic Atrophy | | | | | | | | |
| | Never | | 1.0 | | 0.7(0.3-1.5) | | 0.5(0.1-2.1) | | |
| | Ever | | 2.0 (1.0-3.6) | | 1.3(0.7-2.5) | | 0.9(0.3-3.3) | | |
| | | | | | | | | | |
| | P (Interaction) | | | | 0.96 (CT vs. CC) | | 0.89 (TT vs. CC) | | 0.90 |
| | | | | | | | | | |
| | Neovascular AMD | | | | | | | | |
| | Never | | 1.0 | | 1.3(0.7-2.1) | | 0.6(0.2-1.5) | | |
| | Ever | | 2.2 (1.4-3.5) | | 1.8(1.1-2.9) | | 0.7(0.3-1.9) | | |
| | | | | | | | | | |
| | P (Interaction) | | | | 0.21 (CT vs. CC) | | 0.38 (TT vs. CC) | | 0.25 |
| | | | | | | | | | |
| | | | | | | | | | |
| | | | | | | | | | |
| Lutein: | Advanced AMD | | | | | | | | |
| calorie adjusted, | ≤ mean | | 1.0 | | 0.8(0.5-1.3) | | 0.7(0.3-1.7) | | |
| sex specific | > mean | | 0.7(0.4-1.1) | | 0.7(0.4-1.1) | | 0.2(0.1-0.5) | | |
| | | | | | | | | | |
| | P (Interaction) | | | | 0.62(CT vs. CC) | | 0.11 (TT vs. CC) | | 0.83 |
| | | | | | | | | | |
| | | | | | | | | | |
| | Geographic Atrophy | | | | | | | | |
| | ≤ mean | | 1.0 | | 0.7(0.3-1.2) | | 1.0(0.3-3.1) | | |
| | > mean | | 0.6(0.4-1.2) | | 0.5(0.2-0.9) | | 0.1(0.0-0.7) | | |
| | | | | | | | | | |
| | P (Interaction) | | | | 0.77(CT vs. CC) | | 0.067(TT vs. CC) | | 0.48 |
| | | | | | | | | | |
| | Neovascular AMD | | | | | | | | |
| | ≤ mean | | 1.0 | | 0.9(0.5-1.4) | | 0.6(0.2-1.7) | | |
| | > mean | | 0.7(0.5-1.2) | | 0.8(0.5-1.3) | | 0.2(0.1-0.6) | | |
| | 3P (Interaction) | | | | 0.50(CT vs. CC0 | | 0.30(TT vs. CC) | | 0.88 |
| | | | | | | | | | |
| | | | | | | | | | |
| | | | | | | | | | |
| OR=Odds Ratio, CI= 95% Confidence Interval | | | | | | | | | |
| Mean Lutein= 1355 µg | | | | | | | | | |

## Claims

1. A method for determining age-related macular degeneration (AMD) risk in a patient, comprising: detecting in a patient sample the presence of a cytidine polymorphism at rs493258 and/or a thymine polymorphism at **rs10468017,** wherein the presence of a cytidine polymorphism at rs493258 and/or a thymine polymorphism at rs10468017 is indicative of AMD risk in the patient.

2. The method of Claim 1, comprising combining genetic risk with behavioral risk.

3. The method of claim 1 or 2, wherein the presence of a particular allele is detected by a hybridization assay.

4. The method of claim 1 or 2, wherein the presence of a particular allele is determined using a microarray.

5. The method of claim 1 or 2, wherein the presence of a particular allele is determined using an antibody.

6. The method of claim 1 or 2, wherein the method comprises detecting the presence of a haplotype, wherein the haplotype comprises:
(a) polymorphic site rs493258; or
(b) polymorphic site rs10468017.

7. The method of claim 2,wherein a behavioral risk is assessed by determining if the subject exhibits a behavior or trait selected from the group consisting of: obesity, smoking, vitamin and dietary supplement intake, use of alcohol or drugs, poor diet and a sedentary lifestyle, and optionally wherein elevated BMI is used to determine obesity.

## Patentansprüche

1. Verfahren zum Ermitteln des Risikos altersbedingter Makuladegeneration (AMD) bei einem Patienten, umfassend: Detektieren des Vorhandenseins eines Cytidin-Polymorphismus bei rs493258 und/oder eines Thymin-Polymorphismus bei rs10468017 in einer Patientenprobe, wobei das Vorhandensein eines Cytidin-Polymorphismus bei rs493258 und/oder eines Thymin-Polymorphismus bei rs10468017 ein AMD-Risiko bei dem Patienten anzeigt.

2. Verfahren gemäß Anspruch 1, dasein Kombinieren von genetischem Risiko mit Verhaltensrisiko umfasst.

3. Verfahren gemäß Anspruch 1 oder 2, wobei das Vorhandensein eines bestimmten Allels durch einen Hybridisierungsassay detektiert wird.

4. Verfahren gemäß Anspruch 1 oder 2, wobei das Vorhandensein eines bestimmten Allels mit einem Microarray ermittelt wird.

5. Verfahren gemäß Anspruch 1 oder 2, wobei das Vorhandensein eines bestimmten Allels mit einem Antikörper ermittelt wird.

6. Verfahren gemäß Anspruch 1 oder 2, wobei das Verfahren ein Detektieren des Vorhandenseins eines Haplotyps umfasst, wobei derHaplotyp umfasst:
(a) polymorphe Stelle rs493258 oder
(b) polymorphe Stelle rs10468017.

7. Verfahren gemäß Anspruch 2, wobei ein Verhaltensrisiko durch Ermitteln dessen beurteilt wird, ob das Individuum ein Verhalten oder Merkmal zeigt, das aus der Gruppe ausgewählt ist, welche aus Fettleibigkeit, Rauchen, Einnahme von Vitaminen und Nahrungszusätzen, Konsum von Alkohol oder Drogen, schlechter Ernährung und einer sitzenden Lebensweise besteht, und wobei optional ein erhöhter Körperrrtasseindex (BMI) zum Ermitteln von Fettleibigkeit verwendet wird.

## Revendications

1. Méthode de détermination d'un risque de dégénérescence maculaire liée à l'âge (DMLA) chez un patient, comprenant: la détection chez un prélèvement de patient de la présence d'un polymorphisme de cytidine sur rs493258 et/ou d'un polymorphisme de thymine sur rs10468017, dans lequel la présence d'un polymorphisme de cytidine sur rs493258 et/ou d'un polymorphisme de thymine sur rs10468017 est indicatrice de risque de DMLA chez le patient.

2. Méthode selon la revendication 1, comprenant la combinaison d'un risque génétique avec un risque comportemental.

3. Méthode selon la revendication 1 ou 2, dans laquelle la présence d'un allèle particulier est détectée par une analyse d'hybridation.

4. Méthode selon la revendication 1 ou 2, dans laquelle la présence d'un allèle particulier est détectée en utilisant une microzone.

5. Méthode selon la revendication 1 ou 2, dans laquelle la présence d'un allèle particulier est déterminée en utilisant un anticorps.

6. Méthode selon la revendication 1 ou 2, dans laquelle la méthode comprend la détection de la présence d'un halotype, dans laquelle l'halotype comprend :
(a) un site récepteur polymorphe rs493258 ; ou
(b) un site récepteur polymorphe rs10468017.

7. Méthode selon la revendication 2, dans laquelle un risque comportemental est évalué en déterminant si le sujet montre un comportement ou un trait sélectionné à partir du groupe se composant de: l'obésité, le tabagisme, la prise de vitamines et de compléments alimentaires, la consommation d'alcool ou de médicaments, un régime alimentaire insuffisant et un style de vie sédentaire, et en option dans laquelle un IMC élevé est utilisé pour déterminer l'obésité.
